# EUROPEAN PATENT APPLICATION

(11) **EP 4 818 212 A1**
(43) Date of publication of application: **07.10.2026**
(21) Application number: 24896346.4
(22) Date of filing: 19.11.2024
(51) Int. Cl.: C07K 19/00, A61P 37/02, A61P 31/00, C12N 15/62, C12N 15/85, C12N 5/10, A61K 47/68, A61K 38/20, A61K 47/65, A61K 45/06, A61P 35/00

(54) **BIFUNCTIONAL COMPOSITE MOLECULE OF ANTI-TUMOR ANTIBODY AND INTERLEUKIN-15 PRECURSOR, AND USE OF BIFUNCTIONAL COMPOSITE MOLECULE**

(30) Priority: 28.11.2023 CN 202311607426
(71) Applicant: Changping National Laboratory, Beijing 102206 (CN)
(72) Inventor: FU, Yangxin, Beijing 102206 (CN); GUO, Jingya, Beijing 102206 (CN)
(74) Representative: Metroconsult Srl
(86) International application number: PCT/CN2024/132991
(87) International publication number: WO 2025/113255

(57) **Abstract**

The present inventions provides a complex molecule, a nucleic acid, a vector, a host cell, and a pharmaceutical composition, and use thereof in the manufacture of a medicament for treating cancer. The complex molecule comprises an antitumor antibody domain, a linker, and pro-IL-15, wherein the antitumor antibody domain and pro-IL-15 are linked by the linker. The antitumor antibody domain is a complete antibody, a nanobody, or an antigen-binding fragment thereof against an immune checkpoint molecule, a tumor antigen molecule, or an immune activation molecule. The linker is a polypeptide linker or a non-peptide linker. The pro-IL-15 is a fusion protein comprising IL-15, an IL-15Rα sushi domain, a linking peptide, and optionally an IL-15Rβ extracellular domain, wherein the IL-15Rβ extracellular domain, IL-15, and the IL-15Rα sushi domain are linked by linking peptides.

## Description

### TECHNICAL FIELD

The present disclosure relates to the field of biomedicine, and particularly to a bifunctional molecule formed by linking pro-IL-15 to an antitumor antibody or an antigen-binding fragment thereof, and use thereof in tumor treatment.

### BACKGROUND ART

Tumor immunotherapy, particularly immune checkpoint blockade (ICB), represents a revolutionary advance in cancer treatment. Immune checkpoints, such as CTLA-4, PD-1, and PD-L1, are signaling molecules expressed on the surface of activated immune cells serving to prevent excessive immune activation. When an immune response becomes excessive, they keep immune cells quiescent and act as a "brake" on the immune response. The tumor microenvironment, however, uses these molecules to evade attack by the immune system. Immune checkpoint blockade agents can remove the suppression of tumor-infiltrating lymphocytes (TILs) by the tumor microenvironment and activate antitumor immune responses. As compared with CTLA-4 blocking antibodies, PD-L1/PD-1 (PD) blocking antibodies have attracted substantial attention since they have less adverse effects and more pronounced therapeutic efficacy. Clinically, 10-20% of patients with malignant tumors have seen remarkable therapeutic effects after treatment with PD-blocking agents. Nevertheless, a large proportion of patients have failed to respond to such agents, or relapsed after an initial response. Although PD-blocking agents can partially restore T lymphocyte functionality in tumors, these cells may still differentiate into an exhausted state, thereby reducing or losing their antitumor function.

T-cell growth factors have become important immunomodulatory molecules in tumor immunotherapy because of their ability to expand T lymphocytes. IL-2 was among the earliest cytokine agents approved by the U.S. FDA for malignant melanoma and renal cancer. However, IL-2 has not been widely used in clinical practice, mainly for three reasons: (1) it activates regulatory T cells (Tregs) having an immunosuppressive function; (2) the activated effector T cells are eliminated through activation-induced cell death (AICD); and (3) because IL-2 receptors are widely expressed on vascular endothelial cells, IL-2 treatment may cause severe toxic side effects. IL-15 is another important T-cell growth factor, and is a pleiotropic cytokine capable of inducing and maintaining innate and adaptive immune responses. IL-15 can promote activation, proliferation, and survival of CD8⁺ T cells; induce the generation of long-term memory T cells and maintain their homeostatic proliferation; induce and expand NK and NKT cells; and, in an autocrine manner, promote activation, proliferation, and differentiation of dendritic cells (DCs), enhance the expression of MHC-II and CD80/CD86, and improve the ability of DCs to cross-present antigens. As compared with IL-2, IL-15 has the advantages in that (1) it does not induce AICD; and (2) it does not expand Treg cells. Studies have shown that IL-15 is a component of the inflammatory environment in tumor tissue and is necessary for tumors to establish a normal level of infiltrating CD8⁺ T cells. For example, IL-15 deficiency in patients with colorectal cancer is closely associated with reduced T-cell proliferation, tumor recurrence, and decreased patient survival.

However, the clinical application of IL-15 still faces significant challenges. That is because most cytokine receptors are widely expressed on cells in peripheral tissues, and systemically injected cytokines on one hand cause severe toxicity by acting on peripheral cells, and on the other hand are consumed or eliminated in peripheral tissues such that their concentration in the tumor is insufficient to activate intratumoral immune cells, thereby resulting in suboptimal therapeutic efficacy. The foregoing represents common challenges and bottlenecks encountered in the clinical application of cytokine agents. Current clinical and preclinical studies focus primarily on development of IL-15 fusion proteins, such as the IL-15/IL-15Rα complex, or screening for IL-15 mutants to prolong the half-life of IL-15 and improve the biological activity of the agent, with the ultimate goal of improving antitumor efficacy. However, because IL-15R is mainly expressed on NK cells, NKT cells, and T cells, an increased affinity of IL-15 for its receptor causes extensive consumption of IL-15 before it enters the tumor due to its high-affinity binding to peripheral immune cells, and also increases IL-15-induced toxicity. It has been found that severe toxic side effects caused by IL-15 therapy are primarily mediated by extensive expansion of peripheral NK cells^{1,2}.

To reduce toxicity and improve antitumor efficacy by unleashing IL-15's activity only in tumor tissue, the inventors previously designed pro-IL-15 that releases active IL-15 specifically in tumor tissue, and comprises sIL-15 (a fusion protein of IL-15 and the IL-15Rα sushi domain), an MMP-cleavable peptide, and an IL-15Rβ extracellular domain². Because MMPs (matrix metalloproteinases) are highly expressed specifically in tumor tissue but expressed at a low level in normal tissue, pro-IL-15 remains in a masked state in the periphery to prevent toxicity caused by release of IL-15, but after it enters tumor tissue and is cleaved at the linking peptide by MMP-14 which is highly expressed in tumors, it releases IL-15 to activate antitumor immune responses. Previous studies showed that tumor-responsive pro-IL-15 maintains an antitumor effect comparable to that of IL-15 while significantly reducing toxicity, and can also expand T lymphocytes in tumor tissue, especially stem cell-like lymphocytes with a self-renewal and differentiation potential (TCF1⁺Tim3⁻CD8⁺T cells).

### SUMMARY OF INVENTION

Although pro-IL-15 has been found to effectively expand CD8⁺ T cells in tumor tissue, it remains unsolved how IL-15 can be delivered specifically to antigen-specific CD8⁺ T cells within a tumor so that IL-15 can more effectively expand CD8⁺ CTLs and in turn restore, reverse, or enhance the effector function of CD8⁺ CTLs.

Current clinical and preclinical studies mainly use tumor-antigen-targeting antibodies or polypeptides to deliver IL-15 to tumors. These strategies, however, focus only on delivering cytokines to a tumor site, rather than to effector CD8⁺ T cells. In addition, most cytokines exhibit a receptor-binding affinity much higher than the antigen-antibody binding affinity. As a result, these strategies may lead to cytokine-driven behavior, thereby impairing antibody-mediated targeting.

After extensive research, the present inventors unexpectedly found that a complex of the tumor-responsive pro-IL-15 and an antitumor antibody domain (for example, a complete antibody or an antigen-binding fragment thereof against an immune checkpoint molecule, a tumor antigen molecule, or an immune activation molecule, preferably a PD-blocking antibody, more preferably a PD-1 antibody) enables targeted delivery of pro-IL-15 and allows the antitumor antibody and IL-15 to exert their antitumor functions synergistically. For example, when the antitumor antibody is a PD-1 antibody, a complex molecule of the anti-PD-1 antibody and pro-IL-15 responsive to PD-1⁺CTLs in tumors may be constructed and has the following effects: (1) the activity of the complex molecule is masked before it enters a tumor, thereby avoiding severe toxicity; (2) the complex molecule specifically targets and acts on tumor-infiltrating PD1⁺CD8⁺ CTLs; (3) after reaching the target site, the PD-1 antibody removes the tumor microenvironment-mediated suppression of CD8⁺ CTLs and restores the effector function of CD8⁺ T cells; and (4) the released IL-15 expands CD8⁺ CTLs *in situ*, increases the population and effector function of CD8⁺ CTLs, and induces generation of long-term memory immune cells, thereby breaking the immune tolerance in the tumor microenvironment, increasing tumor treatment efficacy, and preventing recurrence.

Accordingly, in a first aspect, the present disclosure provides a complex molecule comprising an antitumor antibody domain, a linker, and pro-IL-15, wherein the antitumor antibody domain and pro-IL-15 are linked by the linker; the antitumor antibody domain is a complete antibody, a nanobody, or an antigen-binding fragment thereof against an immune checkpoint molecule, a tumor antigen molecule, or an immune activation molecule; the linker is a polypeptide linker or a non-peptide linker; the pro-IL-15 is a fusion protein comprising, or consisting of, an IL-15Rβ extracellular domain, IL-15, an IL-15Rα sushi domain, a first linking peptide, and a second linking peptide, wherein the IL-15Rβ extracellular domain, IL-15, and the IL-15Rα sushi domain are linked by the first linking peptide and the second linking peptide; or the pro-IL-15 is a fusion protein comprising, or consisting of, IL-15, an IL-15Rα sushi domain, and a first linking peptide, wherein IL-15 and the IL-15Rα sushi domain are optionally linked by the first linking peptide.

In the complex molecule (for example, anti-PD-1-pro-IL-15), the pro-IL-15 may be (i) a fusion protein consisting of, from the N-terminus to the C-terminus, an IL-15Rβ extracellular domain (RβD1), a first linking peptide (for example, an MMP-cleavable peptide), IL-15, a second linking peptide, and an IL-15Rα sushi domain; (ii) a fusion protein consisting of, from the N-terminus to the C-terminus, an IL-15Rα sushi domain, a second linking peptide, IL-15, a first linking peptide (for example, an MMP-cleavable peptide), and an IL-15Rβ extracellular domain (RβD1); (iii) a fusion protein consisting of, from the N-terminus to the C-terminus, IL-15, a first linking peptide (for example, an MMP-cleavable peptide), and an IL-15Rα sushi domain; or (iv) a fusion protein consisting of, from the N-terminus to the C-terminus, a first linking peptide (an MMP-cleavable peptide), IL-15, and an IL-15Rα sushi domain. In the case of (iv), the linker may be the first linking peptide *per se* (for example, an MMP-cleavable peptide), that is, the antitumor antibody domain and the pro-IL-15 are linked by the first linking peptide (for example, an MMP-cleavable peptide). The first linking peptide may be an MMP-cleavable peptide or a non-enzymatically cleavable peptide, and the second linking peptide may be a non-enzymatically cleavable peptide.

In certain embodiments of the complex molecule according to the present disclosure, the immune checkpoint molecule may include PD-1, PD-L1, CTLA4, TIGIT, Tim3, or Lag3; the tumor antigen molecule may include CLDN18.2, EGFR, Her2, or mesothelin; and the immune activation molecule may include OX40, 4-1BB, CD28, or CD3; but not limited thereto.

In certain embodiments of the complex molecule according to the present disclosure, the antitumor antibody domain may be a PD-1 antibody or an antigen-binding fragment thereof; the antigen-binding fragment may include an ScFv, Fab, or F(ab')₂.

In certain embodiments of the complex molecule according to the present disclosure, the complex molecule may be a fusion protein, the linker may be a polypeptide, and the complex molecule may be in a form selected from:
form (a) wherein the C-terminus of at least one heavy chain of the complete antibody or nanobody is linked to the N-terminus of a pro-IL-15 via a linker;
form (b) wherein the C-terminus of an antigen-binding fragment is linked to the N-terminus of a pro-IL-15 via a linker; or
form (c) wherein the N-terminus of one chain of an Fc region is linked, via a linker, to the C-terminus of a fusion protein in form (b); and the N-terminus of the other chain of the Fc region is not linked to a peptide, or is linked, via a linker, to the C-terminus of a fusion protein in form (b), to the C-terminus of an antigen-binding fragment, or to the C-terminus of a pro-IL-15;
wherein in forms (b) and (c), the C-terminus of an antigen-binding fragment refers to the C-terminus of the heavy chain or light chain of a Fab, the C-terminus of an scFv, or the C-terminus of one heavy chain or light chain of an F(ab')₂; wherein, when multiple linkers are present in the complex molecule, the linkers may be the same as or different from each other; and when two pro-IL-15 are present in the complex molecule, the two pro-IL-15 may be the same as or different from each other.

In certain embodiments of the complex molecule according to the present disclosure, the complete antibody or nanobody may be a bispecific antibody.

In certain embodiments of the complex molecule, preferably the linker has an amino acid sequence of SEQ ID No: 8 or SEQ ID No: 10.

In certain embodiments of the complex molecule according to the present disclosure, the complex molecule may be a conjugate, and the linker may be a non-peptide linker.

In certain embodiments of the complex molecule according to the present disclosure, the linker may be an enzymatically cleavable linker.

In certain embodiments of the complex molecule according to the present disclosure, the linker may be a non-enzymatically cleavable linker.

In certain embodiments of the complex molecule according to the present disclosure, the two chains of the Fc region of the antitumor antibody may be linked to pro-IL-15 molecules having different sequences, with the two linkers the same as or different from each other.

In certain embodiments of the complex molecule according to the present disclosure, the complex molecule may be in the above-mentioned form (a), in which the C-terminus of at least one heavy chain of a complete antibody or nanobody is linked to the N-terminus of pro-IL-15 via a linker. In this case, the sequence of at least one heavy chain of the complex molecule (i.e., the peptide chain formed by linking the at least one heavy chain of a complete antibody or nanobody to pro-IL-15) may consist of, from the N-terminus to the C-terminus, at least one of the following: (1) a heavy chain of a Fab of an antitumor antibody, an Fc, a linker, an Rα sushi domain, a second linking peptide, IL-15, a first linking peptide, and RβD1; (2) a heavy chain of a Fab of an antitumor antibody, an Fc, a linker, RβD1, a first linking peptide, IL-15, a second linking peptide, and an Rα sushi domain; (3) a heavy chain of a Fab of an antitumor antibody, an Fc, a linker, IL-15, a first linking peptide, and an Rα sushi domain; and (4) a heavy chain of a Fab of an antitumor antibody, an Fc, a first linking peptide, IL-15, and an Rα sushi domain; wherein the antitumor antibody is an anti-PD-1 antibody, an anti-Lag3 antibody, or an anti-Claudin18.2 antibody; wherein each heavy chain of the Fab is associated with a corresponding antibody light chain; and wherein the linker is a non-enzymatically cleavable linker, the first linking peptide is an MMP-cleavable peptide or a non-enzymatically cleavable peptide, and the second linking peptide is a non-enzymatically cleavable peptide.

In certain embodiments of the complex molecule according to the present disclosure, when an Fc is included, the Fc may be a wild-type Fc, or may be preferably an engineered Fc (for example, a mutated Fc) having reduced or abolished binding affinity to FcγR, or may be an engineered Fc (for example, a mutated Fc) having enhanced binding to FcγR. Preferably, the Fc region may have no ADCC function (mFc); for example, the amino acid sequence of the Fc region may be SEQ ID No: 3, and the Fc region may further comprise an L235E mutation.

In certain embodiments of the complex molecule according to the present disclosure, the IL-15 in the pro-IL-15 may be a mammalian wild-type IL-15 (for example, SEQ ID No: 6 or 16), or may be one of the N71Q, N72D, N79Q, and N112Q mutants thereof.

In certain embodiments of the complex molecule according to the present disclosure, the antitumor antibody may be a complete antitumor antibody or a Fab fragment thereof, and the Fab may be at least one selected from: (i) a Fab having a heavy chain sequence of SEQ ID No: 2 or SEQ ID No: 12, and a light chain sequence of SEQ ID No: 1 or SEQ ID No: 11; (ii) a Fab having a heavy chain sequence of SEQ ID No: 19, and a light chain sequence of SEQ ID No: 18; or (iii) a Fab having a heavy chain sequence of SEQ ID No: 21, and a light chain sequence of SEQ ID No: 20.

In certain embodiments of the complex molecule according to the present disclosure, in the pro-IL-15, the sequence of IL-15 may be SEQ ID No: 6 or SEQ ID No: 16, the sequence of the IL-15Rα sushi domain may be SEQ ID No: 7 or SEQ ID No: 17, the sequence of the IL-15Rβ extracellular domain may be SEQ ID No: 4, SEQ ID No: 14, SEQ ID No: 5, or SEQ ID No: 15, the sequence of the first linking peptide may be SEQ ID No: 8, 9, or 10, and the sequence of the second linking peptide may be SEQ ID No: 8 or SEQ ID No: 9.

The present disclosure further provides a complex molecule obtained by inserting pro-IL-15 or IL-15 between the CH1 region and the CH2 region of at least one heavy chain of a complete antitumor antibody, wherein linkers are optionally linked to the N-terminus and C-terminus of the pro-IL-15 or IL-15, wherein the pro-IL-15 is a fusion protein consisting of, from the N-terminus to the C-terminus, IL-15, a linking peptide, and an IL-15Rα sushi domain, or an IL-15Rα sushi domain, a linking peptide, and IL-15; wherein the antitumor antibody, linkers, and linking peptide may be the same as those described in the above embodiments.

In certain embodiments of the complex molecule according to the present disclosure, the complex molecule is a fusion protein in a form in which one heavy chain consists of, from the N-terminus to the C-terminus, a heavy chain of a Fab of an antitumor antibody, a linker, pro-IL-15/IL-15, a linker, and one chain of an Fc region; the other heavy chain consists of, from the N-terminus to the C-terminus, a heavy chain of a Fab of an antitumor antibody, a linker, pro-IL-15/IL-15, a linker, and the other chain of the Fc region; wherein the two Fab heavy chains are the same as or different from each other and each of them is associated with a corresponding antibody light chain, for example through a disulfide bond, to form a Fab; the two chains of the Fc are associated with each other through a disulfide bond; the linkers are the same as or different from one another; and the two pro-IL-15 are the same as or different from each other. Preferably, the Fab is selected from a Fab of an anti-PD-1 antibody, an anti-Lag3 antibody, or an anti-Claudin 18.2 antibody; the linker is selected from SEQ ID No: 8, SEQ ID No: 9, or SEQ ID No: 10; the IL-15 is selected from SEQ ID No: 6 or SEQ ID No: 16; the IL-15Rα sushi domain is selected from SEQ ID No: 7 or SEQ ID No: 17; the linking peptide is selected from SEQ ID No: 8 or SEQ ID No: 9; the Fc may be a wild-type Fc, or may be preferably an engineered Fc (for example, mutated) having reduced or abolished binding to FcγR, or may be an engineered Fc (for example, mutated) having enhanced binding to FcγR. For example, the amino acid sequence of the Fc region may be SEQ ID No: 3, and the Fc region may further comprise an L235E mutation. More preferably, the linker connected to the Fab heavy chain is SEQ ID No: 8 or SEQ ID No: 9, and the linker connected to the Fc is SEQ ID No: 10.

When the complex molecule according to the present disclosure is a fusion protein, the present disclosure further provides a nucleic acid encoding any of the fusion proteins described above, a vector comprising the nucleic acid, and a host cell comprising the vector.

In a second aspect, the present disclosure provides a pharmaceutical composition comprising the complex molecule, nucleic acid, vector, or host cell according to the first aspect of the present disclosure, and a pharmaceutically acceptable carrier or excipient. In certain embodiments of the second aspect, the antitumor antibody domain may be a complete antibody, a nanobody, or an antigen-binding fragment thereof against PD-1, and the pharmaceutical composition may further comprise an anti-PD-L1 antibody.

In a third aspect, the present disclosure provides use of the complex molecule, nucleic acid, vector, or host cell according to the first aspect of the present disclosure, or use of the pharmaceutical composition according to the second aspect of the present disclosure, in the manufacture of a medicament for treating cancer, preferably a solid tumor.

In a fourth aspect, the present disclosure provides a method for treating cancer, comprising administering to a subject an effective amount of the complex molecule according to the first aspect of the present disclosure or the pharmaceutical composition according to the second aspect of the present disclosure; preferably, wherein the subject is a mammal, more preferably a human.

The cancer described in the third aspect or the fourth aspect includes, but is not limited to: lung cancer, liver cancer, gastric cancer, cervical cancer, ovarian cancer, breast cancer, colorectal cancer, melanoma, and lymphoma.

### BRIEF DESCRIPTION OF DRAWINGS

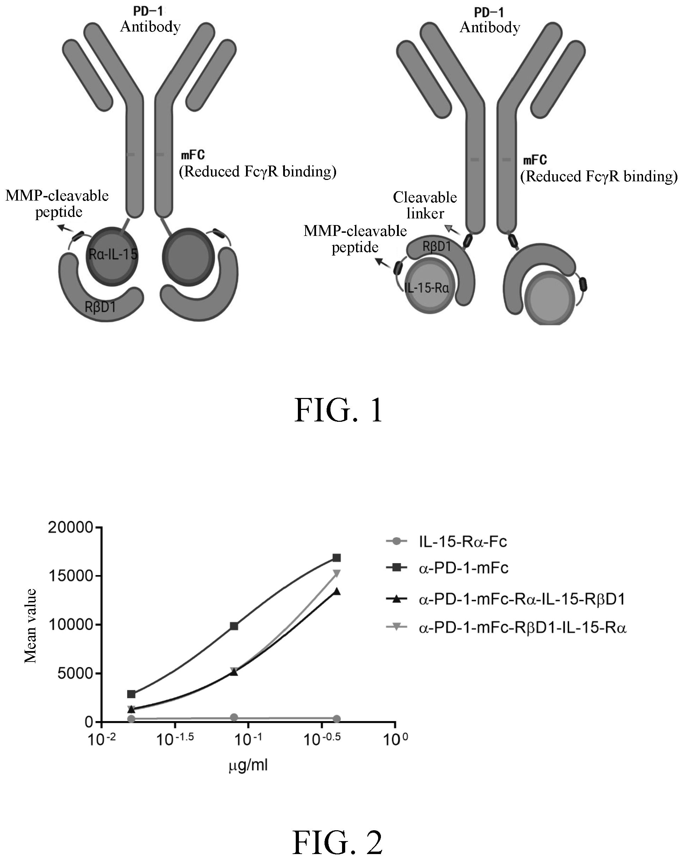

FIG. 1 schematically illustrates two examples of a complex molecule anti-PD-1-pro-IL-15 according to the present disclosure. The left panel shows a non-separable anti-PD-1-pro-IL-15 fusion protein, in which the "Rα-IL-15" represents a fusion molecule consisting of, from the N-terminus to the C-terminus, an Rα sushi domain (Rα), a non-enzymatically cleavable linking peptide, and IL-15. The right panel shows a separable anti-PD-1-pro-IL-15 fusion protein, in which the "IL-15-Rα" represents a fusion molecule consisting of, from the N-terminus to the C-terminus, IL-15, a non-enzymatically cleavable linking peptide, and an Rα sushi domain.
FIG. 2 shows the binding ability of the proteins used in Example 2 to cell surface PD-1: IL-15-Rα linked only to an Fc region (IL-15-Rα-Fc); a PD-1 antibody alone (α-PD-1-mFc); a non-separable anti-PD-1-pro-IL-15 fusion protein (α-PD-1-mFc-Rα-IL-15-RβD1); and a separable anti-PD-1-pro-IL-15 fusion protein (α-PD-1-mFc-RβD1-IL-15-Rα).
FIG. 3 shows the bioactivity of IL-15 in two IL-15-Rα fusion proteins linked to an Fc region only (i.e. Fc-Rα-IL-15 and IL-15-Rα-Fc), and in the two anti-PD-1-pro-IL-15 fusion proteins used in Example 2.
FIG. 4 shows the gel electrophoresis of two anti-PD-1-pro-IL-15 fusion proteins before and after MMP14 treatment.
FIG. 5 shows the bioactivity of IL-15 in two anti-PD-1-pro-IL-15 fusion proteins before and after MMP14 treatment.
FIG. 6 shows the therapeutic efficacy of two anti-PD-1-pro-IL-15 fusion proteins, a blank control (CTR), and an unmasked anti-PD-1-mFc-Rα-IL-15 fusion protein (without RβD1) in a mouse MC38 tumor model.
FIG. 7 shows the therapeutic efficacy of a blank control (CTR), a non-separable anti-PD-1-pro-IL-15 fusion protein (α-PD-1-mFc-Rα-IL-15-RβD1), and a physical mixture of a PD-1 antibody and pro-IL-15 (α-PD-1-mFc + pro-IL-15) in a mouse MC38 tumor model. *** indicates p<0.001.
FIG. 8 shows the antitumor efficacy of an anti-PD-1-pro-IL-15 fusion protein (α-PD-1-mFc-Rα-IL-15-RβD1) after blockade of IFN-γ, depletion of CD8⁺ T cells, or depletion of NK cells.
FIG. 9 shows changes in antigen-specific CD8⁺ T cells and stem cell-like T cells in tumor after treatment with anti-PD-1-pro-IL-15.
FIG. 10 shows tumor inhibition by a combination of anti-PD-1-pro-IL-15 and a PD-L1 antibody.
FIG. 11 shows the results of size-exclusion chromatography coupled with multi-angle static light scattering (SEC-MALS) for the different protein molecules prepared in Example 8.
FIG. 12 shows the binding ability of the fusion proteins prepared in Example 8 to cell-surface human PD-1 (hPD-1): human IgG1 (hIgG1); an anti-PD-1 antibody alone (αPD-1); an anti-PD-1 antibody linked to sIL-15 only (αPD-1-sIL-15); the human-derived fusion protein αPD-1-pro-IL-15(RβD1); and the human-derived fusion protein αPD-1-pro-IL-15(Rβ).
FIG. 13 shows gel electrophoresis for two human-derived fusion proteins prepared in Example 8 and their cleavage products after MMP14 treatment. (A): lanes 1 and 2 are PD-1 antibody-Rα-IL-15-Rβ, and lane 3 is PD-1 antibody-Rα-IL-15. (B): lanes 1 and 2 are PD-1 antibody-Rα-IL-15-RβD1, lane 3 is PD-1 antibody-Rα-IL-15-RβD1 after MMP14 treatment, and lane 4 is PD-1 antibody-Rα-IL-15.
FIG. 14 shows the IL-15 bioactivity of two human-derived fusion proteins prepared in Example 8 and their cleavage products after MMP14 treatment on (A) NK cells and (B) CD8⁺ T cells.
FIG. 15 shows the therapeutic efficacy of two human-derived fusion proteins prepared in Example 8, a blank control (CTR), and a physical mixture of the PD-1 antibody and pro-IL-15 (proIL-15 + α-PD-1) against CFPAC1 tumors in a CBMC humanized mouse model. * indicates p<0.05; ** indicates p<0.01; *** indicates p<0.001.
FIG. 16 shows a schematic representation of a physically masked fusion protein anti-PD-1-Fab-IL-15-Rα-mFc.
FIG. 17 shows the mass photometry results of the isolated and purified physically masked fusion proteins anti-PD-1-Fab-IL-15-Rα-mFc and anti-PD-1-Fab-IL-15-mFc prepared in Example 9.
FIG. 18 shows the binding ability of the physically masked fusion proteins prepared in Example 9 to PD-1: anti-PD-1 antibody alone (αPD-1), anti-PD-1-Fab-IL-15-Rα-mFc, anti-PD-1-Fab-IL-15-mFc, and human IgG1 (hIgG1).
FIG. 19 shows the binding ability of IL-15 or sIL-15 in the physically masked fusion proteins prepared in Example 9 to the IL-15β receptor CD122.
FIG. 20 shows the bioactivity of the physically masked fusion proteins anti-PD-1-Fab-IL-15-Rα-mFc and anti-PD-1-Fab-IL-15-mFc prepared in Example 9 measured in a CTLL-2 proliferation assay.
FIG. 21 shows the antitumor efficacy and toxic side effects of the physically masked fusion proteins anti-PD-1-Fab-IL-15-Rα-mFc and anti-PD-1-Fab-IL-15-mFc prepared in Example 9 in a mouse MC38 tumor model. *** indicates p<0.001; **** indicates p<0.0001.
FIG. 22 shows the antitumor efficacy of the fusion protein anti-Lag3-pro-IL-15 prepared in Example 10 in a mouse MC38 tumor model. **** indicates p<0.0001.
FIG. 23 shows the bioactivity of IL-15 in two anti-Claudin18.2-pro-IL-15 fusion proteins prepared in Example 11.
FIG. 24 shows the antitumor efficacy of the fusion protein anti-Claudin18.2-Fc-RβD1-IL-15-Rα prepared in Example 11 in a mouse MC38 tumor model. **** indicates p<0.0001.

### DESCRIPTION OF EMBODIMENTS

Exemplary embodiments of the present disclosure are described in detail below. However, it should be understood that the exemplary embodiments described below are provided only to illustrate the principles of the present invention and are not intended to limit the scope of the present invention.

Unless otherwise specified, the terms "Rα sushi domain", "Rα", and "sushi" used herein are used interchangeably and refer to the sushi domain of the Rα receptor of IL-15. Its sequence may be SEQ ID No: 7 or SEQ ID No: 17, or a mutant sequence thereof having an equivalent function.

Unless otherwise specified, the term "IL-15Rβ extracellular domain" used herein refers to the extracellular domain of the Rβ receptor of IL-15 or a truncated functional fragment thereof (for example, "IL-15Rβ extracellular domain 1" or "RβD1"). Its sequence may be SEQ ID No: 4, SEQ ID No: 14, SEQ ID No: 5, or SEQ ID No: 15, or a mutant sequence thereof having an equivalent function.

Unless otherwise specified, the term "antitumor antibody domain" as used herein refers to a complete antibody, nanobody, or antigen-binding fragment thereof that specifically binds a tumor-associated molecule and exerts an antitumor effect including, but not limited to, inhibiting tumor occurrence, progression, and metastasis. The tumor-associated molecule may include an immune checkpoint molecule, such as PD-1, PD-L1, CTLA4, TIGIT, Tim3, or Lag3, a tumor antigen molecule such as CLDN18.2, EGFR, Her2, or mesothelin, or an immune activation molecule such as OX40, 4-1BB, CD28, or CD3, but not limited thereto. A "complete antibody" refers to an antibody having the conventional complete structure of two heavy chains and two light chains. The term "nanobody" refers to an antibody that, compared to a complete antibody, has only two heavy chains and no light chains, with the heavy chains lacking CH1. The antigen-binding fragment may include an ScFv, Fab, or F(ab')₂ fragment of a complete antibody, or may include a Fab or F(ab')₂ fragment of a nanobody.

The term "treatment" used herein generally refers to obtaining a desired pharmacological and/or physiological effect that partially or completely stabilizes or cures a disease and/or adverse effects caused by the disease, or inhibits or alleviates symptoms of the disease.

The term "effective amount" as used herein refers to an amount of the complex molecule or pharmaceutical composition according to the present disclosure administered to achieve therapeutic efficacy. A "therapeutically effective amount" may vary depending on, for example, the disease state, age, gender, and weight of the individual, as well as the ability of the therapeutic agent to elicit a desired response in the subject.

The term "IL-15" as used herein refers to interleukin-15, a cytokine of the interleukin family, including mammalian-derived IL-15, such as mouse-derived or human-derived IL-15.

The present disclosure provides a complex molecule comprising an antitumor antibody domain, a linker, and pro-IL-15, wherein the antitumor antibody domain and pro-IL-15 are linked via the linker. The antitumor antibody domain is a complete antibody, a nanobody, or an antigen-binding fragment thereof against an immune checkpoint molecule, a tumor antigen molecule, or an immune activation molecule; and the linker is a polypeptide linker or a non-peptide linker. The pro-IL-15 is a fusion protein comprising an IL-15Rβ extracellular domain, IL-15, an IL-15Rα sushi domain, a first linking peptide, and a second linking peptide, wherein the IL-15Rβ extracellular domain, IL-15, and IL-15Rα sushi domain are connected by the first and second linking peptides. Alternatively, the pro-IL-15 is a fusion protein comprising IL-15, an IL-15Rα sushi domain, and a first linking peptide, wherein the IL-15 and the IL-15Rα sushi domain are optionally connected by the first linking peptide. Unless otherwise specified, the order in which the functional units of pro-IL-15, i.e. IL-15Rβ extracellular domain, IL-15, and IL-15Rα sushi domain are connected is not limited.

In certain embodiments, the present disclosure provides a complex molecule formed by linking an antitumor antibody domain (for example, a PD blocking antibody) and pro-IL-15 via a linker. The complex molecule specifically targets and acts on tumor-infiltrating PD1⁺CD8⁺ CTL cells. After the complex molecule reaches a target site, the PD-1 antibody thereof removes the suppression of CD8⁺ CTL cells by the tumor microenvironment and restores the effector function of the CD8⁺ T cells, while IL-15 is released to expand CD8⁺ CTLs *in situ*, increases both the effector function and the number of CD8⁺ CTLs, and induces long-term memory immune cells. The complex molecule according to the present disclosure can break immune tolerance in the tumor microenvironment, improve the tumor treatment efficacy, and prevent recurrence.

In certain embodiments of the complex molecule according to the present disclosure, the PD blocking antibody may be a PD-1 antibody or a PD-L1 antibody, and may be a monoclonal antibody known in the art. For example, the heavy chain of a Fab of the PD-1 antibody has a sequence of SEQ ID No: 2 or SEQ ID No: 12, and the light chain thereof has a sequence of SEQ ID No: 1 or SEQ ID No: 11, but not limited thereto.

In certain embodiments of the complex molecule according to the present disclosure, the Lag3 antibody may be a monoclonal antibody known in the art. For example, the heavy chain of a Fab of the Lag3 antibody preferably has a sequence of SEQ ID No: 19, and the light chain thereof has sequence of SEQ ID No: 18, but not limited thereto.

In certain embodiments of the complex molecule according to the present disclosure, the Claudin18.2 (CLDN18.2) antibody may be a monoclonal antibody known in the art. For example, the heavy chain of a Fab of the Claudin18.2 antibody preferably has a sequence of SEQ ID No: 21, and the light chain thereof has a sequence of SEQ ID No: 20, but not limited thereto.

In certain embodiments of the complex molecule according to the present disclosure, pro-IL-15 is a fusion protein and may be: (i) a fusion protein consisting of, from the N-terminus to the C-terminus, an IL-15Rβ extracellular domain (for example, RβD1), a first linking peptide (for example, an MMP-cleavable peptide), IL-15, a second linking peptide, and an IL-15Rα sushi domain; (ii) a fusion protein consisting of, from the N-terminus to the C-terminus, an IL-15Rα sushi domain, a second linking peptide, IL-15, a first linking peptide (for example, an MMP-cleavable peptide), and an IL-15Rβ extracellular domain (for example, RβD1); (iii) a fusion protein consisting of, from the N-terminus to the C-terminus, IL-15, a first linking peptide (for example, an MMP-cleavable peptide), and an IL-15Rα sushi domain; or (iv) a fusion protein consisting of, from the N-terminus to the C-terminus, a first linking peptide (for example, an MMP-cleavable peptide), IL-15, and an IL-15Rα sushi domain. In the case of (iv), the linker may be the first linking peptide *per se* (for example, an MMP-cleavable peptide). The first linking peptide may be an MMP-cleavable peptide or a non-enzymatically cleavable peptide, and the second linking peptide may be a non-enzymatically cleavable peptide.

The activity of IL-15 in the complex molecule may be masked before the molecule enters a tumor, and thus no severe toxic side effects are caused. After the complex molecule enters a tumor, MMP14, which is highly expressed in tumors, can cleave the MMP-cleavable peptide serving as the first linking peptide, thereby releasing IL-15 to exert its function. The second linking peptide linking IL-15 and the IL-15Rα sushi domain in the pro-IL-15, if present, may be non-enzymatically cleavable.

In certain embodiments of the complex molecule according to the present disclosure, the linker connecting the antitumor antibody domain (for example, a PD blocking antibody) and pro-IL-15 may be a polypeptide linker or a non-peptide linker. Examples of a polypeptide linker include SEQ ID Nos: 8, 9, and 10.

In certain embodiments of the complex molecule according to the present disclosure, the complex molecule may be a fusion protein, the linker may be a polypeptide, and the complex molecule may be in a form selected from:
form (a) wherein the C-terminus of at least one heavy chain of the complete antibody or nanobody is linked to the N-terminus of a pro-IL-15 via a linker;
form (b) wherein the C-terminus of an antigen-binding fragment is linked to the N-terminus of a pro-IL-15 via a linker;
form (c) wherein the N-terminus of one chain of an Fc region is linked, via a linker, to the C-terminus of a fusion protein in form (b), and the N-terminus of the other chain of the Fc region is not linked to a peptide, or is linked, via a linker, to the C-terminus of a fusion protein in form (b), to the C-terminus of an antigen-binding fragment, or to the C-terminus of a pro-IL-15;
wherein in forms (b) and (c), the C-terminus of the antigen-binding fragment refers to the C-terminus of the heavy chain or light chain of a Fab, the C-terminus of an scFv, or the C-terminus of one heavy chain or light chain of an F(ab')₂; wherein, when multiple linkers are present in the complex molecule, the linkers may be the same as or different from each other; and when two pro-IL-15 are present in the complex molecule, the two pro-IL-15 may be the same as or different from each other.

In certain embodiments of the complex molecule according to the present disclosure, the complex molecule is obtained by inserting pro-IL-15 or IL-15 between the CH1 and CH2 of at least one heavy chain of a complete antitumor antibody, wherein a linker is optionally linked to the N-terminus and C-terminus of the pro-IL-15 or IL-15. In this case the pro-IL-15 is a fusion protein consisting of, from the N-terminus to the C-terminus, IL-15, a linking peptide, and an IL-15Rα sushi domain; or an IL-15Rα sushi domain, a linking peptide, and IL-15. The antitumor antibody, linker, and linking peptide may be the same as those described above.

In certain embodiments of the complex molecule according to the present disclosure, the complex molecule is a fusion protein in a form in which one heavy chain consists of, from the N-terminus to the C-terminus, a heavy chain of a Fab of an antitumor antibody, a linker, pro-IL-15/IL-15, a linker, and one chain of an Fc region; and the other heavy chain consists of, from the N-terminus to the C-terminus, a heavy chain of a Fab of an antitumor antibody, a linker, pro-IL-15/IL-15, a linker, and the other chain of the Fc region; wherein the two Fab heavy chains are the same as or different from each other and each of them is associated with a corresponding antibody light chain, for example through a disulfide bond, to form a Fab; the two chains of the Fc are associated with each other through a disulfide bond; the linkers are the same as or different from one another; and the two pro-IL-15 are the same as or different from each other. Preferably, the Fab is selected from a Fab of an anti-PD-1 antibody, an anti-Lag3 antibody, or an anti-Claudin 18.2 antibody; the linker is selected from SEQ ID No: 8, SEQ ID No: 9, or SEQ ID No: 10; the IL-15 is selected from SEQ ID No: 6 or SEQ ID No: 16; the IL-15Rα sushi domain is selected from SEQ ID No: 7 or SEQ ID No: 17; the linking peptide is selected from SEQ ID No: 8 or SEQ ID No: 9; the Fc may be a wild-type Fc, or may be an engineered Fc (for example, mutated) having reduced or abolished binding to FcγR, or may be an engineered Fc (for example, mutated) having enhanced binding to FcγR. Preferably, the Fc region may have no ADCC function (mFc); for example, the amino acid sequence of the Fc region may be SEQ ID No: 3, and the Fc region may further comprise an L235E mutation. More preferably, the linker connected to the Fab heavy chain is SEQ ID No: 8 or SEQ ID No: 9, and the linker connected to the Fc is SEQ ID No: 10.

In certain embodiments of the complex molecule according to the present disclosure, the linker may be enzymatically cleavable or non-enzymatically cleavable, preferably non-enzymatically cleavable. The inventors have found that, when the linker is non-enzymatically cleavable, the antitumor antibody domain (for example, a PD-blocking antibody) and pro-IL-15 are substantially non-separable both in the periphery and in tumors, and unexpectedly exhibit better antitumor efficacy.

In addition, the complex molecule according to the present disclosure may be a molecule in which the pro-IL-15 lacks an IL-15Rβ extracellular domain (for example, RβD1). For example, it may comprise a pro-IL-15 of (iii) or (iv) described above. The inventors have found that, in the absence of RβD1, the activity of the complex molecule "(anti-PD-1-Fc)-linker-(IL-15)-(IL-15Rα sushi)" is reduced relative to sIL-15; however, when the complex molecule adopts (iii) or (iv), for example "(anti-PD-1-Fc)-linker-(IL-15)-MMP-cleavable peptide-(IL-15Rα sushi)" or "(anti-PD-1-Fc)-linker-MMP-cleavable peptide-(IL-15)-(IL-15Rα sushi)," the activity is restored to a certain extent.

Furthermore, both the first and second linking peptides in the pro-IL-15 of the complex molecule according to the present disclosure may be non-enzymatically cleavable peptides. In an individual pro-IL-15 molecule, the first linking peptide is typically an MMP-cleavable peptide, thereby ensuring efficient release of IL-15 in the tumor environment to exert its function. However, the inventors unexpectedly found that, when pro-IL-15 is linked to an antibody (for example, to the Fc of a PD-1 antibody), even if both the first and second linking peptides in pro-IL-15 are non-enzymatically cleavable peptides, the complex molecule can still exhibit IL-15 activity significantly higher than that of the corresponding pro-IL-15 not linked to the antibody (when the cells simultaneously express PD-1 molecules and IL-15 receptors).

In a specific embodiment, the complex molecule according to the present disclosure is a fusion protein shown in the left panel of FIG. 1, and the functional units of the fusion protein include, from the N-terminus to the C-terminus, an anti-PD-1 Fab, mFc (having reduced binding to FcγR), a linker, an Rα sushi domain, a linking peptide, IL-15, an MMP-cleavable peptide, and RβD1, wherein the linker is a non-enzymatically cleavable polypeptide linker, rendering the antibody and pro-IL-15 in the complex molecule substantially non-separable *in vivo* (i.e., a "non-separable" complex molecule). The term "non-separable" as used herein is not limited to the complex molecule of this embodiment. Any complex molecule in which the antigen-binding portion of the antibody and pro-IL-15 are linked by a non-enzymatically cleavable linker may be referred to as "non-separable".

In a specific embodiment, the complex molecule according to the present disclosure is the fusion protein shown in the right panel of FIG. 1, and the functional units of the fusion protein include, from the N-terminus to the C-terminus, an anti-PD-1 Fab, mFc (with reduced binding to FcγR), a linker, RβD1, an MMP-cleavable peptide, IL-15, a linking peptide, and an Rα sushi domain, wherein the linker is an enzymatically cleavable polypeptide linker, for example an MMP-cleavable peptide. Therefore, the antibody portion and pro-IL-15 of the complex molecule are separated from each other after entering a tumor due to cleavage of the linker (i.e., a "separable" complex molecule). It should be understood that the term "separable" as used herein is not limited to the complex molecule of this embodiment. Any complex molecule in which the antigen-binding portion of the antibody and pro-IL-15 are connected by an enzymatically cleavable linker may be referred to as "separable."

The structure of the complex molecule according to the present disclosure is not limited to the above embodiments. For example, different types of pro-IL-15 molecules may be linked to the two Fc chains of an antibody. In addition, the non-enzymatically cleavable linker in the left panel of FIG. 1 may be replaced with an enzymatically cleavable linker to provide a separable complex molecule, and the enzymatically cleavable linker in the right panel of FIG. 1 may be replaced with a non-enzymatically cleavable linker to provide a non-separable complex molecule.

When the complex molecule according to the present disclosure is a fusion protein, the present disclosure further provides a nucleic acid encoding any of the fusion proteins described above, a vector comprising the nucleic acid, and a host cell comprising the vector.

The present disclosure further provides a pharmaceutical composition comprising the complex molecule, nucleic acid, vector, or host cell according to the present disclosure, and a pharmaceutically acceptable carrier or excipient. In one embodiment, the pharmaceutical composition may further comprise a second active agent, for example another anticancer agent (for example, a small molecule anticancer agent or an antibody anticancer agent). In one embodiment, the pharmaceutical composition is for use in treatment of a tumor or cancer.

The present disclosure further provides use of the complex molecule, nucleic acid, vector, host cell, or pharmaceutical composition according to the present disclosure in the manufacture of a medicament for treating cancer, preferably a solid tumor.

The present disclosure further provides a method for treating cancer, preferably a solid tumor, comprising administering to a subject an effective amount of the complex molecule or pharmaceutical composition according to the present disclosure; wherein the subject is preferably a mammal, more preferably a human.

The tumor or cancer mentioned above includes, but is not limited to, lung cancer, liver cancer, gastric cancer, cervical cancer, ovarian cancer, breast cancer, colorectal cancer, melanoma, and lymphoma.

### Example

The following experiments are provided as examples to illustrate the technical concept of the present disclosure or to demonstrate the effects of the present disclosure. It should be understood, however, that the present disclosure is not limited to these examples, and various equivalent substitutions or modifications may be made without departing from the technical concept of the present disclosure.

### Materials and Methods

### Construction of Fusion Proteins

The pEE12.4-IgGκ plasmid was from the inventors' lab and contained a mouse IgGκ signal peptide. The Fab sequence of the PD-1 antibody was the J43 clone capable of blocking PD-L1/PD-1 signaling. The Fc sequence was a human IgG1 Fc sequence lacking an ADCC function, as set forth in SEQ ID NO: 3. IL-15-related genes (including IL-15 SEQ ID No: 6, IL-15Rα SEQ ID No: 7, IL-15Rβ extracellular end SEQ ID No: 5, and IL-15Rβ extracellular domain 1 SEQ ID No: 4) were all synthesized by Shanghai Bioin Biological Technology Co., Ltd., fused to the anti-PD-1 antibody sequence, and cloned into the pEE12.4 expression vector. Plasmids were extracted using a plasmid maxi-prep kit (Tiangen) and stored at -80°C.

### Laboratory Animals

Wild-type C57BL/6 mice were purchased from Beijing Vital River Laboratory Animal Technology Co., Ltd., China. Unless otherwise specified, all experiments were performed using 8- to 10-week-old female mice. All mice were bred in a specific pathogen-free (SPF) housing facility. Animal breeding and experimental procedures complied with the regulations of the Laboratory Animal Management Committee.

### Cell Lines

**FreeStyle^{™} 293F** (Invitrogen) suspension cells were derived from the HEK293 cell line, cultured in SMM293-TII or CD OptiCHO^{™} medium, and mainly used for transient transfection to express fusion proteins.

**CTLL-2**, a mouse T cell line, was used to detect the biological activity of IL-15. The cells were cultured in RPMI 1640 complete medium (containing 10% inactivated fetal bovine serum, 2 mmol/L L-glutamine, 0.1 mmol/L non-essential amino acids, 100 U penicillin, 100 µg/ml streptomycin, 100 IU/ml recombinant IL2, and 55 µM β-mercaptoethanol).

**CTLL-2-mPD-1**, a cell line stably expressing mouse PD-1 molecules, was cultured in RPMI 1640 complete medium (containing 10% inactivated fetal bovine serum, 2 mmol/L L-glutamine, 0.1 mmol/L non-essential amino acids, 100 U penicillin, 100 µg/ml streptomycin, 400 IU/ml recombinant IL2, 55 µM β-mercaptoethanol, and 4 µg/ml puromycin).

**MC38**, a C57-background mouse colorectal cancer cell line, was cultured in DMEM complete medium (containing 10% inactivated fetal bovine serum, 2 mmol/l L-glutamine, 0.1 mmol/l non-essential amino acids, 100 U penicillin, and 100 µg/ml streptomycin).

### Detection of Protein-Binding Ability

Cells expressing a specific antigen were collected and resuspended in a FACS buffer (PBS + 2% FBS + 0.05% sodium azide). Each sample to which 0.2 µg of 2.4G2 antibody was added was incubated on ice for 15 minutes to block FcγR. Fusion proteins were serially diluted in a FACS buffer. The proteins were co-incubated with the cell suspension on ice for 30 minutes. After washing once with a FACS buffer, a fluorescently labeled anti-human IgG1 antibody was added, followed by incubation on ice for 30 minutes. After washing twice with a FACS buffer, the samples were analyzed on the instrument.

### CTLL-2 Proliferation Assay

CTLL-2 cells are a mouse T cell line, and their proliferation can be used to determine the biological activity of IL-15. Fusion proteins or the products after MMP14 cleavage were serially diluted with a complete medium, and 100 µl of each dilution was added to a 96-well plate. CTLL-2 cells or CTLL-2-mPD-1 cells were collected and washed to remove IL-2 from the culture medium. The cell concentration was adjusted to 3 × 10⁴ cells/mL, and 100 µL of the cell suspension was added to each well. After the cells were cultured for 72 hours in a 37°C, 5% CO₂ incubator, 20 µl of the CCK8 reagent was added to each well, followed by further incubation at 37°C and 5% CO₂ for 3-4 hours. OD450 was measured using a microplate reader, and curves were plotted and analyzed.

### Verification of In Vitro Cleavage and Activity Restoration of anti-PD-1-pro-IL-15

An activation buffer (50 mM Tris, 1 mM CaCl₂, 0.5% Brij-35, pH 9.0) and an assay buffer (50 mM Tris, 3 mM CaCl₂, 1 µM ZnCl₂, pH 7.5) were prepared separately and sterile-filtered for use. In the activation buffer, rhMMP14 (R&D) was activated with rhFurin at 37°C. Anti-PD-1-pro-IL-15 was diluted in the assay buffer, and the activated MMP14 was added thereto in a molar ratio of the enzyme to the protein to be cleaved of 1:5, followed by overnight incubation at 37°C. SDS-PAGE was used to assess whether anti-PD-1-pro-IL-15 could be specifically cleaved by MMP14 and to determine the cleavage efficiency. The CTLL-2 proliferation assay was used to evaluate the restoration of activity of anti-PD-1-pro-IL-15 after MMP14 cleavage.

### Establishment of Mouse Tumor Models

MC38 was selected as the research model. Establishment of the tumor model: 2-5×10⁵ MC38 single cells were suspended in 100 µl PBS and subcutaneously inoculated into the right side of the back of C57BL/6 mice. The longest diameter (a), shortest diameter (b), and height (c) of the tumor were measured using a caliper, and the mouse tumor volume was calculated as a×b×c/2. The tumor size was monitored twice weekly, and the tumor growth curves of the mice were plotted.

### Analysis of Peripheral Blood Immune Cells

Orbital blood was collected in anticoagulation tubes containing K2 EDTA. 50 µL of the sample was transferred to a flow cytometry tube. A fluorescent antibody mixture was prepared according to the specific staining protocol and added to the flow cytometry tube, mixed well, and placed at room temperature in the dark for 15 min. 450 µL of the red blood cell lysis buffer (eBioscience 1X RBC Lysis Buffer) was added dropwise to the blood sample under shaking, followed by standing at room temperature in the dark for 15 min. Before flow cytometric analysis, 15 µL of mircobeads (Invitrogen Absolute Counting Beads) was added. Data were collected on a BD FACS Fortessa flow cytometer. The absolute number of each immune cell population in peripheral blood was calculated based on the proportion and number of the mircobeads added.

### Preparation of Single Cell Suspension from Mouse Tumor Tissue

The tumor tissue was taken and added in a 6-well plate containing 100 µl of RPMI 1640 medium; the tumor tissue was thoroughly minced using an ophthalmic scissor, and 2 ml of a digestion solution (RPMI 1640 medium containing 1 mg/ml collagenase and 500 U/ml DNase I) was added to resuspend the tissue fragments. The tissue-fragment suspension was digested on a 37°C shaker at 145 rpm until no visible particles remained (the procedure lasted approximately 40 minutes), with pipetting up and down 20 times every 20 minutes during digestion. The digested tissue suspension was filtered through a 70-mesh cell strainer, and the cells were washed twice with RPMI 1640 medium containing 2% FBS; the cells were resuspended in 1 ml RPMI 1640 complete medium and counted for subsequent use.

### Flow Cytometric Analysis of Immune Cells in Tumor

The tumor single cell suspension was transferred to a flow cytometry tube at 1×10⁶ cells per tube. An Fc receptor-blocking antibody (clone 2.4G2) was added to block Fc receptors on the cell surface, followed by incubation on ice for 20 min. According to the specific staining protocol, a fluorescent antibody mixture was prepared and added to the flow cytometry tube, followed by incubation on ice in the dark for 30 min. A single-stained tube, an isotype control tube, and a blank tube were prepared in parallel. After washing twice with a FACS buffer, the cells were resuspended, and data were collected using a BD FACS Fortessa flow cytometer.

Intracellular protein molecule staining was performed as follows: cells were resuspended by adding 300 µl pre-cooled Foxp3 Fixation/Permeabilization buffer, incubated on ice in the dark for 30-60 min, and resuspended in 1 mL of a permeabilization buffer, followed by centrifugation at 500 g for 3 min, and the supernatant was discarded; the cells were resuspended in 100 µl of a permeabilization buffer, fluorescent antibodies were added, and the cells were incubated on ice in the dark for 30 min; after washing twice with a permeabilization buffer, the cells were resuspended, and data were collected using a BD FACS Fortessa flow cytometer.

### Biostatistical Analysis

All data were analyzed using the statistical analysis software GraphPad Prism. Mouse survival curves were analyzed by the One-way ANOVA, and all other data were analyzed using a two-tailed t-test. Differences were considered statistically significant at P < 0.05 (*), highly significant at P < 0.01 (**), and very highly significant at P < 0.001 (***) or P < 0.0001 (****).

### Sequence Information

SEQ ID No: 1: light chain of anti-mouse PD-1 antibody 
YELTQPPSASVNVGETVKITCSGDQLPKYFADWFHQRSDQTILQVIYDDNKRPSGI
PERISGSSSGTTATLTIRDVRAEDEGDYYCFSGYVDSDSKLYVFGSGTQLTVLGRTVAAP
SVFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC
SEQ ID No: 11: light chain of anti-human PD-1 antibody 
EIVLTQSPATLSLSPGERATLSCRASKGVSTSGYSYLHWYQQKPGQAPRLLIYLASY
LESGVPARFSGSGSGTDFTLTISSLEPEDFAVYYCQHSRDLPLTFGGGTKVEIKRTVAAPS
VFIFPPSDEQLKSGTASVVCLLNNFYPREAKVQWKVDNALQSGNSQESVTEQDSKDST
 YSLSSTLTLSKADYEKHKVYACEVTHQGLSSPVTKSFNRGEC
SEQ ID No: 2: heavy chain of Fab of anti-mouse PD-1 antibody 
EVRLLESGGGLVKPEGSLKLSCVASGFTFSDYFMSWVRQAPGKGLEWVAHIYTKS
YNYATYYSGSVKGRFTISRDDSRSMVYLQMNNLRTEDTATYYCTRDGSGYPSLDFWG
QGTQVTVSSAASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKRV
SEQ ID No: 12: heavy chain of Fab of anti-human PD-1 antibody 
QVQLVQSGVEVKKPGASVKVSCKASGYTFTNYYMYWVRQAPGQGLEWMGGIN
PSNGGTNFNEKFKNRVTLTTDSSTTTAYMELKSLQFDDTAVYYCARRDYRFDMGFDY
WGQGTTVTVSSASTKGPSVFPLAPCSRSTSESTAALGCLVKDYFPEPVTVSWNSGALTS
GVHTFPAVLQSSGLYSLSSVVTVPSSSLGTKTYTCNVDHKPSNTKVDKRV
SEQ ID No: 3: mutant human Fc (L19A, L20A, P114G) 
EPKSCDKTHTCPPCPAPEAAGGPSVFLFPPKPKDTLMISRTPEVTCVVVDVSHEDPE
VKFNWYVDGVEVHNAKTKPREEQYNSTYRVVSVLTVLHQDWLNGKEYKCKVSNKA
LGAPIEKTISKAKGQPREPQVYTLPPSRDELTKNQVSLTCLVKGFYPSDIAVEWESNGQP
ENNYKTTPPVLDSDGSFFLYSKLTVDKSRWQQGNVFSCSVMHEALHNHYTQKSLSLSP
GK
SEQ ID No: 13: human Fc (WT) 
EPKSCDKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYKCKVSNKA
LPAPIEKTISKAKGQPREPQVYTLPPSRDELTKNQVSLTCLVKGFYPSDIAVEWESNGQP
ENNYKTTPPVLDSDGSFFLYSKLTVDKSRWQQGNVFSCSVMHEALHNHYTQKSLSLSP
GK
SEQ ID No: 4: extracellular domain 1 of mouse IL-15Rβ 
AVKNCSHLECFYNSRANVSCMWSHEEALNVTTCHVHAKSNLRHWNKTCELTLV
RQASWACNLILGSFPESQSLTSVDLLDINVVCWEEKGWRRVKTCDF
SEQ ID No: 14: extracellular domain 1 of human IL-15Rβ 
AVNGTSQFTCFYNSRANISCVWSQDGALQDTSCQVHAWPDRRRWNQTCELLPVS
QASWACNLILGAPDSQKLTTVDIVTLRVLCREGVRWRVMAIQDFKPFE
SEQ ID No: 5: extracellular domain of mouse IL-15Rβ AVKNCSHLECFYNSRANVSCMWSHEEALNVTTCHVHAKSNLRHWNKTCELTLV 
RQASWACNLILGSFPESQSLTSVDLLDINVVCWEEKGWRRVKTCDFHPFDNLRLVAPHS
LQVLHIDTQRCNISWKVSQVSHYIEPYLEFEARRRLLGHSWEDASVLSLKQRQQWLFL
EMLIPSTSYEVQVRVKAQRNNTGTWSPWSQPLTFRTRPADPMKE
SEQ ID No: 15: extracellular domain of human IL-15Rβ 
AVNGTSQFTCFYNSRANISCVWSQDGALQDTSCQVHAWPDRRRWNQTCELLPVS
QASWACNLILGAPDSQKLTTVDIVTLRVLCREGVRWRVMAIQDFKPFENLRLMAPISLQ
VVHVETHRCNISWEISQASHYFERHLEFEARTLSPGHTWEEAPLLTLKQKQEWICLETL
TPDTQYEFQVRVKPLQGEFTTWSPWSQPLAFRTKPAALGKDT
SEQ ID No: 6: mouse IL-15 
NWIDVRYDLEKIESLIQSIHIDTTLYTDSDFHPSCKVTAMNCFLLELQVILHEYSNM
TLNETVRNVLYLANSTLSSNKNVAESGCKECEELEEKTFTEFLQSFIRIVQMFINTS
SEQ ID No: 16: human IL-15 
NWVNVISDLKKIEDLIQSMHIDATLYTESDVHPSCKVTAMKCFLLELQVISLESGD
ASIHDTVENLIILANNSLSSNGNVTESGCKECEELEEKNIKEFLQSFVHIVQMFINTS
SEQ ID No: 7: sushi domain of mouse IL-15Rα 
GTTCPPPVSIEHADIRVKNYSVNSRERYVCNSGFKRKAGTSTLIECVINKNTNVAH
WTTPSLKCIRDPSLAHYSPVPT
SEQ ID No: 17: sushi domain of human IL-15Rα 
ITCPPPMSVEHADIWVKSYSLYSRERYICNSGFKRKAGTSSLTECVLNKATNVAHW
TTPSLKCIRDPALVHQRPAPP
SEQ ID No: 8: linking peptide
   GGGGSGGGGSGGGGSGGGGS
SEQ ID No: 9: linking peptide
   SGGGSGGGGSGGGGSGGGGSGGGSLQ
SEQ ID No: 10: MMP-cleavable linking peptide
   GGGGSSGRSPAIFTASGFIANPVTAGGGGS
SEQ ID No: 18: light chain of anti-human/mouse Lag3 antibody 
EIVLTQSPGTLSLSPGERATLSCRASQSVSSYLAWYQQKPGQAPRLLIYGISSRATGI
PDRFSGSGSGTDFTLTISSLQPEDFATYYCQQANNFPLTFGGGTKLEIKRTVAAPSVFIFPP
SDEQLKSGTASVVCLLNNFYPREAKVQWKVDNALQSGNSQESVTEQDSKDSTYSLSST
LTLSKADYEKHKVYACEVTHQGLSSPVTKSFNRGEC
SEQ ID No: 19: heavy chain of Fab of anti-human/mouse Lag3 antibody 
QVQLVQSGAEVKKPGSSVKVSCKASGGTFSSYAISWVRQAPGQGLEWMGGIIPIF
GTANYAQKFQGRVTITADESTSTAYMELSSLRSEDTAVYYCAASTLRVPNPPYWGQGTL
VTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKV
SEQ ID No: 20: light chain of anti-human/mouse Claudin 18.2 antibody 
DIVMTQSPDSLAVSLGERATISCKSSQSLLNSGNQKNYLTWYQQKPGQPPKLLIYW
ASTRESGVPDRFSGSGSGTDFTLTISSLQAEDVAVYYCQNDYFYPFTFGQGTKLEIKRTV
AAPSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC
SEQ ID No: 21: heavy chain of Fab of anti-human/mouse Claudin 18.2 antibody

QVQLVQSGAEVKKPGSSVKVSCKASGYAFSNYLIEWVKQAPGQGLEWIGLINPGS
GGTNYNEKFKGKATITADKSTSTAYMELSSLRSEDTAVYYCARVYYGNSFAYWGQGTL
VTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRV

### Example 1. Construction of Anti-PD-1-pro-IL-15 Fusion Proteins

In this example, two anti-PD-1-pro-IL-15 fusion proteins were designed and constructed. The Fab sequence of the PD-1 antibody (anti-PD-1) was the J43 clone capable of blocking PD-L1/PD-1 signaling; the Fc sequence was a human IgG1 Fc sequence with three mutations L19A, L20A, and P114G, without an ADCC function (designated mFc).

The first fusion protein was a non-separable anti-PD-1-pro-IL-15 fusion protein, a structural schematic representation of which is shown in the left panel of FIG. 1. Its functional units, from the N-terminus to the C-terminus, included an anti-PD-1 Fab (SEQ ID No: 1 and SEQ ID No: 2), mFc (SEQ ID No: 3), a linker (SEQ ID No: 8), an Rα sushi domain (SEQ ID No: 7), a linking peptide (SEQ ID No: 9), IL-15 (SEQ ID No: 6), an MMP-cleavable peptide (SEQ ID No: 10), and RβD1 (SEQ ID No: 4), wherein the linker was a non-enzymatically cleavable polypeptide linker, rendering the antibody and pro-IL-15 in the complex molecule substantially non-separable *in vivo*.

The second fusion protein was a separable anti-PD-1-pro-IL-15 fusion protein, a structural schematic representation of which is shown in the right panel of FIG. 1. Its functional units, from the N-terminus to the C-terminus, included an anti-PD-1 Fab (SEQ ID No: 1 and SEQ ID No: 2), mFc (SEQ ID No: 3), a linker (SEQ ID No: 10), RβD1 (SEQ ID No: 4), an MMP-cleavable peptide (SEQ ID No: 10), IL-15 (SEQ ID No: 6), a linking peptide (SEQ ID No: 9), and an Rα sushi domain (SEQ ID No: 7), wherein the linker was an MMP-cleavable polypeptide. Therefore, the antibody portion and pro-IL-15 of the complex molecule separate from each other under the action of an MMP after the complex molecule enters a tumor.

The corresponding anti-PD-1-Fab heavy chain-pro-IL-15 expression plasmid and anti-PD-1 light chain (SEQ ID No: 1) expression plasmid were constructed using the PEE12.4 eukaryotic expression vector system. The anti-PD-1-Fab heavy chain-pro-IL-15 expression plasmid and the anti-PD-1 light chain expression plasmid were co-transfected into 293F cells to obtain an expression supernatant. The Fc-containing protein was purified by Protein A affinity chromatography, and the protein size was verified by SDS-PAGE.

### Example 2. In Vitro Study of Biological Activity of Anti-PD-1-pro-IL-15

The biological activity of each active unit in the fusion proteins was evaluated *in vitro*.

First, flow cytometry was used to evaluate the binding ability of the PD-1 antibody in the fusion proteins prepared in Example 1 to its antigen. An antibody not linked to pro-IL-15 (anti-PD-1) and a pro-IL-15-Fc not linked to an antibody Fab were used as controls. The results showed that the anti-PD-1-pro-IL-15 and anti-PD-1 were bound to PD-1 on the surface of EG7 cells with similar affinity (FIG. 2).

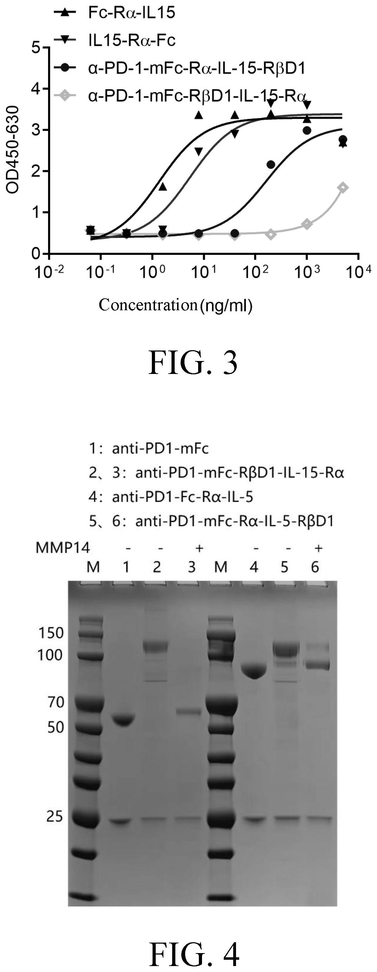

The biological activity of IL-15 in the fusion proteins was evaluated by a CTLL-2 proliferation assay. The results showed that, as compared with unmasked IL-15-Rα-Fc or Fc-Rα-IL-15, the IL-15 activity of the two anti-PD-1-pro-IL-15 fusion proteins according to the present disclosure in an MMP-free environment was markedly reduced, indicating that the IL-15 activity in the fusion proteins can be masked by the receptor (FIG. 3).

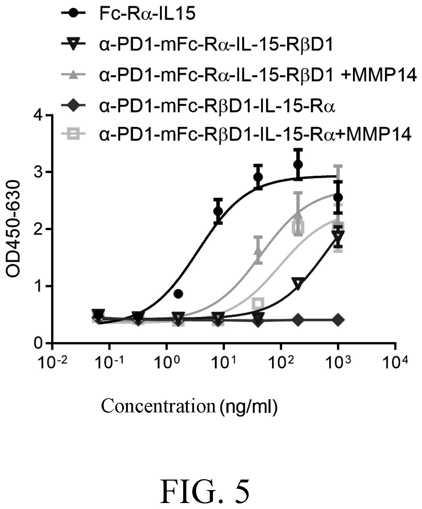

The fusion proteins were co-incubated *in vitro* with activated MMP14 enzyme, and then subjected to SDS-PAGE analysis to verify the *in vitro* cleavage of the fusion proteins. The results showed that the fusion proteins can be effectively cleaved *in vitro* (FIG. 4). Recovery of biological activity after the enzymatic cleavage of the fusion proteins was evaluated by a CTLL-2 proliferation assay. The results showed that, after *in vitro* enzymatic cleavage, the biological activity of IL-15 in the fusion proteins was effectively restored (FIG. 5). The above results indicate that the IL-15 activity of the fusion proteins can be effectively blocked by the IL-15 receptor and can be restored after MMP cleavage.

### Example 3. Non-Separable Anti-PD-1-pro-IL-15 Exhibited Better Antitumor Effect than Separable Anti-PD-1-pro-IL-15

After the function of the anti-PD-1-pro-IL-15 fusion proteins was verified *in vitro*, the antitumor effects and toxic side effects of these proteins were evaluated in a mouse MC38 tumor model. PBS was used as a blank control (CTR); and unmasked anti-PD-1-mFc-Rα-IL-15 (without RβD1) was used as an isotype control. C57BL/6 mice were subcutaneously inoculated on the back with 5×10⁵ MC38 tumor cells. On days 8 and 11, the mice were intravenously injected with 80 µg of either of the anti-PD-1-pro-IL-15 fusion proteins or 70 µg of the control protein anti-PD-1-mFc-Rα-IL-15. The tumor size, body weight, and survival of the mice were monitored at regular intervals. The tumor growth curves and body-weight change curves were plotted.

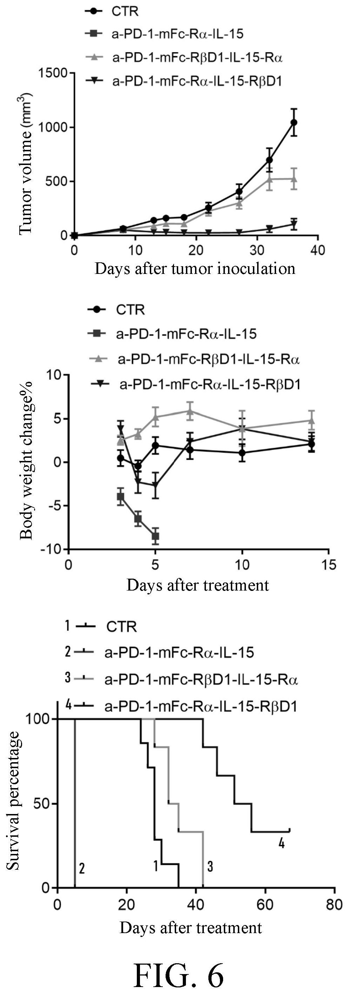

As shown in FIG. 6, after systemic administration, mice treated with unmasked anti-PD-1-mFc-Rα-IL-15 showed a marked decrease in body weight, and all mice in this group died from toxicity on the second day after the second administration. In contrast, no death occurred in either of the two anti-PD-1-pro-IL-15 fusion protein treatment groups, and no significant decrease in body weight was observed. In addition, as compared with separable anti-PD-1-pro-IL-15, the non-separable fusion protein exhibited a better antitumor effect.

The non-separable anti-PD-1-pro-IL-15 fusion protein (α-PD-1-mFc-Rα-IL-15-RβD1) was used in subsequent examples.

### Example 4. Anti-PD-1-pro-IL-15 Exhibited Superior Antitumor Effect Compared with Physical Mixture of PD-1 Antibody and pro-IL-15

A mouse tumor model was used to evaluate whether the antitumor effect of the tumor-targeting anti-PD-1-pro-IL-15 fusion protein was superior to that of a physical mixture of a PD-1 antibody and non-targeting pro-IL-15. PBS was used as a blank control (CTR). C57BL/6 mice were subcutaneously inoculated on the back with 5×10⁵ MC38 tumor cells. On days 8 and 11, the mice were intravenously injected with 80 µg of the non-separable anti-PD-1-pro-IL-15 fusion protein or a physical mixture of 53 µg of the PD-1 antibody and 45 µg of the pro-IL-15. The tumor growth in the mice was monitored at regular intervals.

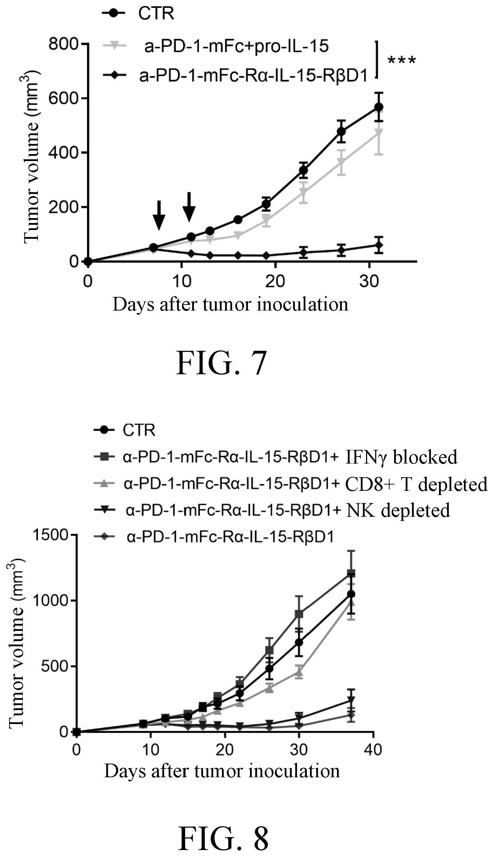

As shown in FIG. 7, upon systemic administration, the anti-PD-1-pro-IL-15 exhibited a significantly better antitumor effect than the physical mixture of the PD-1 antibody and pro-IL-15. On day 31, as compared with the blank control, the physical mixture reduced the tumor volume by approximately 17%, without statistic significance, while the fusion protein reduced the tumor volume by 90% or more, with statistic significance. This result indicates that the antitumor effect of the fusion protein according to the present disclosure does not merely come from the combined effects of the PD-1 antibody and the pro-IL-15, and the delivery of both the PD-1 antibody and pro-IL-15 to the same target site is crucial for tumor inhibition.

### Example 5. In Vivo Antitumor Activity of Anti-PD-1-pro-IL-15 Depends on T Cells and IFN-γ

To investigate the mechanism underlying the antitumor activity of the fusion protein, depleting antibodies were used to systemically deplete NK cells and CD8⁺ T cells, respectively, and a blocking antibody was used to block IFN-γ activity. T cell and NK cell depletion efficiency was evaluated by FACS. The results showed that the depleting antibodies effectively depleted CD8⁺ T cells and NK cells from peripheral blood. C57BL/6 mice were subcutaneously inoculated on the back with 5×10⁵ MC38 tumor cells. On days 9 and 12, the mice were intravenously injected with 80 µg of the anti-PD-1-pro-IL-15. On days 9, 12, 15, and 18, the mice were intraperitoneally injected with 200 µg of the CD8 T-cell-depleting antibody (clone TIB210, prepared in the inventors' lab), or 400 µg of the NK-cell-depleting antibody (clone PK136, prepared in the inventors' lab), or 500 µg of the IFN-γ-blocking antibody (clone R4-6A2). The tumor growth in the mice was monitored at regular intervals.

As shown in FIG. 8, depletion of NK cells during treatment with the fusion protein did not affect its antitumor activity. However, depletion of CD8⁺ T cells completely abolished the antitumor activity. After IFN-γ signaling was blocked with the neutralizing antibody, the same result was observed: the fusion protein completely failed to control the tumor growth. These results indicate that the *in vivo* antitumor effect of the anti-PD-1-pro-IL-15 depends on T cells and IFN-γ.

### Example 6. Differentiation and Conversion of Different Subsets of CD8⁺ T Cells After Anti-PD-1-pro-IL-15 Treatment

C57BL/6 mice were subcutaneously inoculated on the back with 5×10⁵ MC38-OVA tumor cells. The mice were injected with 80 µg of the anti-PD-1-pro-IL-15 on days 8 and 11. On day 14, the numbers of Tetramer-positive antigen-specific CD8⁺T cells and TCF⁺ Tim3⁻CD8⁺T cells in the tumor tissue were measured by FACS.

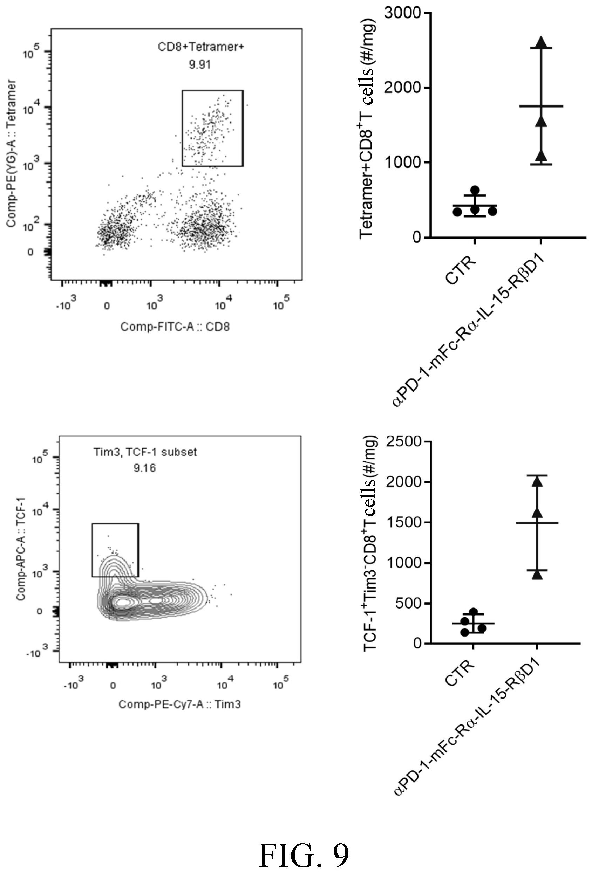

The results showed that, after treatment with the anti-PD-1-pro-IL-15, the numbers of antigen-specific CD8⁺ T cells (OT-I Tetramer⁺CD8⁺T cells) and stem-like CD8⁺ T cells (OT-I Tetramer⁺Tim3⁻PD1⁺TCF-1⁺CD8⁺T cells) in the tumor tissue were significantly increased (FIG. 9). The above results demonstrate that the anti-PD-1-pro-IL-15 fusion protein according to the present disclosure can increase the numbers of antigen-specific CD8⁺ T cells and stem cell-like CD8⁺ T cells in tumors, thereby leading to a good prognosis after immunotherapeutic efficacy.

### Example 7. Anti-PD-1-pro-IL-15 and Anti-PD-L1 Antibody Produced Synergistic Antitumor Effect

Previous experiments showed that the therapeutic effect of the fusion proteins according to the present disclosure depends on IFN-γ. This cytokine upregulates PD-L1 expression, which may further limit the therapeutic efficacy of the fusion protein against tumors. C57BL/6 mice were subcutaneously inoculated on the back with 5×10⁵ MC38 tumor cells, then the mice were intraperitoneally injected with 100 µg of an anti-PD-L1 antibody on days 10 and 13, and intravenously injected with 80 µg of the anti-PD-1-pro-IL-15 on the same days. The tumor growth in the mice was monitored at regular intervals.

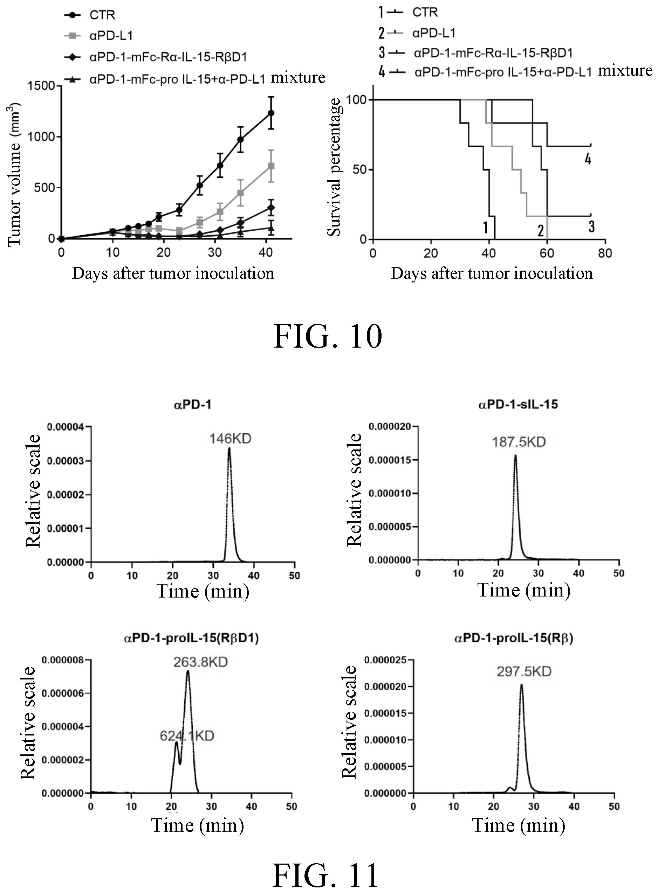

As shown in FIG. 10, combining the anti-PD-1-pro-IL-15 with the anti-PD-L1 antibody produced a much stronger effect than the anti-PD-1-pro-IL-15 or anti-PD-L1 antibody alone. This finding indicates that the anti-PD-L1 antibody can further improve the therapeutic effect of the fusion proteins according to the present disclosure and overcome immune tolerance.

The above results show that the anti-PD-1-pro-IL-15 fusion protein according to the present disclosure can mask the activity of IL-15 in a non-tumor environment, and after it enters a tumor, can release IL-15 and restore IL-15 activity under the action of an MMP enzyme. When the PD-1 antibody and pro-IL-15 are linked by a non-enzymatically cleavable peptide, the fusion protein exhibits a better antitumor effect. The anti-PD-1-pro-IL-15 fusion proteins according to the present disclosure can increase the numbers of antigen-specific CD8⁺ T cells and stem cell-like CD8⁺ T cells in tumors; exhibit an antitumor effect greater than the sum of the effects achieved by separate administration of the PD-1 antibody and pro-IL-15; depend on T cells and IFN-γ to exert the antitumor activity; and can further enhance the antitumor effect by combination with a PD-L1 antibody.

### Example 8. Construction of Human-derived anti-PD-1-pro-IL-15 Fusion Proteins

**8.1** In this example, two human-derived anti-PD-1-pro-IL-15(RβD1 or Rβ) were designed and constructed. The Fab sequence of the PD-1 antibody (anti-PD-1) was the sequence of pembrolizumab capable of blocking PD-L1/PD-1 signaling. The Fc sequence was a human IgG1 Fc sequence with three mutations L19A, L20A, and P114G, and lacked an ADCC function (designated mFc). The pro-IL-15, from the N-terminus to the C-terminus, was "Rα-linking peptide-IL-15-MMP cleavable peptide-RβD1/Rβ". A structural schematic representation of these proteins is shown in the left panel of FIG. 1. Specifically, the functional units of the fusion proteins, from the N-terminus to the C-terminus, include an anti-PD-1 Fab (SEQ ID No: 11 and SEQ ID No: 12), mFc (SEQ ID No: 3), a linker (SEQ ID No: 8), an Rα sushi domain (SEQ ID No: 17), a linking peptide (SEQ ID No: 9), IL-15 (SEQ ID No: 16), an MMP-cleavable peptide (SEQ ID No: 10), and the IL-15Rβ extracellular domain Rβ (SEQ ID No: 15) or IL-15Rβ extracellular domain truncation RβD1 (SEQ ID No: 14). The linker (SEQ ID No: 8) was a non-enzymatically cleavable polypeptide linker, rendering the antibody and pro-IL-15 in the complex molecule substantially non-separable *in vivo*. Therefore, the two human-derived fusion proteins in this example were non-separable fusion proteins.

The corresponding anti-PD-1-Fab heavy chain-pro-IL-15 expression plasmid and the anti-PD-1 light chain (SEQ ID No: 11) expression plasmid were constructed using the PEE12.4 eukaryotic expression vector system. The anti-PD-1-Fab heavy chain-pro-IL-15 expression plasmid and the anti-PD-1 light chain expression plasmid were co-transfected into 293F cells to obtain an expression supernatant. The Fc-containing protein was purified by Protein A affinity chromatography, and the protein size was verified by SDS-PAGE. Protein quality was evaluated by SEC-MALS (size-exclusion chromatography and static light scattering) (FIG. 11). As shown in FIG. 11, in the SEC-MALS spectrum, the fusion protein with a molecular weight of 263.8 kD is anti-PD-1-pro-IL-15(RβD1), and the fusion protein with a molecular weight of 297.5 kD is anti-PD-1-pro-IL-15(Rβ).

### 8.2 In Vitro Biological Activity Study of Human-derived anti-PD-1-pro-IL-15

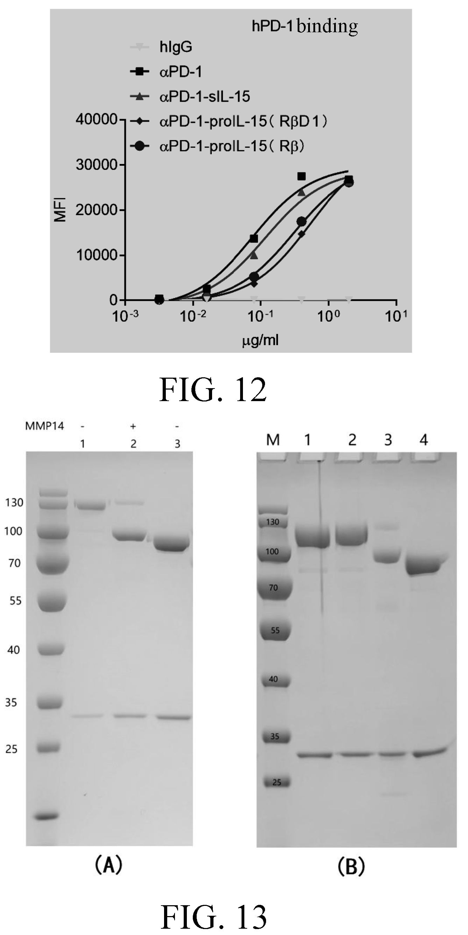

Flow cytometry was used to evaluate the binding ability of the PD-1 antibody portions in the two human-derived fusion proteins prepared in **8.1** to human PD-1 on the surface of CTLL-2 cells (CTLL-2-hPD-1 cells). An anti-PD-1 antibody having the same sequence as that in the above fusion proteins and not linked to a pro-IL-15 (αPD-1), an anti-PD-1 antibody having the same sequence as that in the above fusion proteins and linked only to sIL-15 (i.e. IL-15-Rα sushi) (αPD-1-sIL-15), and human IgG1 (hIgG1) were used as controls. The results showed that the two human-derived anti-PD-1-pro-IL-15 fusion proteins were bound to PD-1 with a similar affinity to that of the anti-PD-1 antibody (FIG. 12).

The two human-derived fusion proteins were co-incubated *in vitro* with activated MMP14 and then subjected to SDS-PAGE analysis to verify the *in vitro* cleavage of the fusion proteins. The results showed that both human-derived fusion proteins were effectively cleaved *in vitro* (FIG. 13). In FIG. 13(A), lanes 1 and 2 were PD-1 antibody-Rα-IL-15-Rβ, and lane 3 was PD-1 antibody-Rα-IL-15. In FIG. 13(B), lanes 1 and 2 were PD-1 antibody-Rα-IL-15-RβD1, lane 3 was PD-1 antibody-Rα-IL-15-RβD1 after MMP14 treatment, and lane 4 was PD-1 antibody-Rα-IL-15.

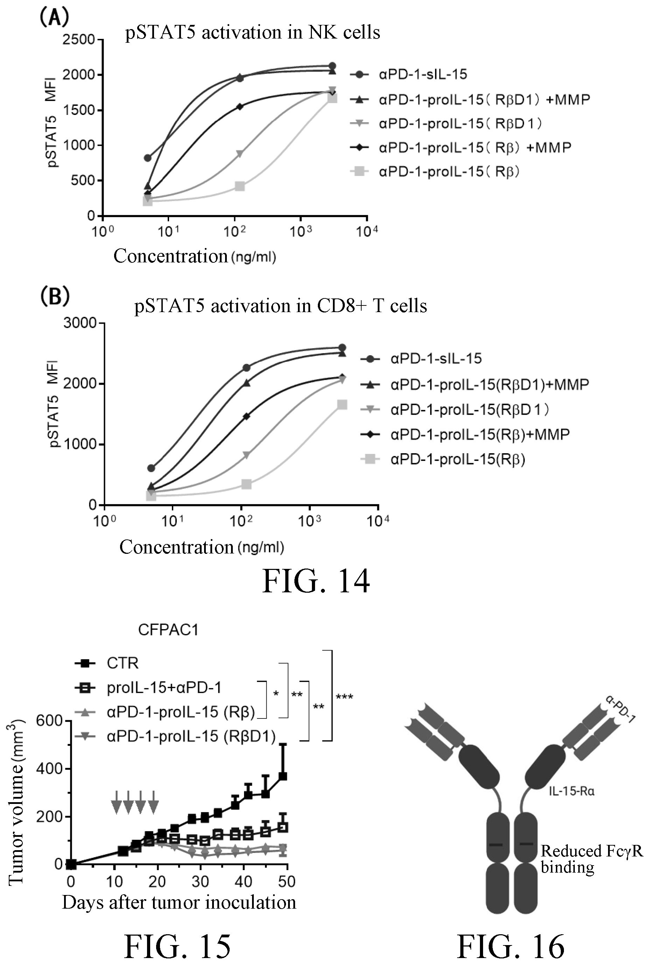

Restoration of biological activity after the enzymatic cleavage of the fusion proteins was evaluated by a STAT5 phosphorylation assay in NK cells and CD8⁺ T cells in PBMCs. The results showed that, after the *in vitro* enzymatic cleavage, the biological activity of IL-15 in the fusion proteins was effectively restored (FIG. 14). The above results indicate that IL-15 activity of the fusion proteins can be effectively blocked by the IL-15 receptor and well restored after MMP cleavage.

### 8.3 Human-derived Anti-PD-1-pro-IL-15 Effectively Controlled Tumor Growth in Humanized Mouse Model

The antitumor effects of the two human-derived anti-PD-1-pro-IL-15 fusion proteins prepared in 8.1 were tested in a humanized mouse model. NSG mice were subcutaneously inoculated with 1×10⁶ CFPAC1 cells. On day 10 after tumor-cell inoculation, the mice were intravenously injected with 7×10⁶ human CBMC cells. On days 12, 15, 18, and 21, the mice received intraperitoneal injections of 80 µg of the anti-PD-1-pro-IL-15(RβD1), or 90 µg of the anti-PD-1-pro-IL-15(Rβ), or a mixture of 40 µg of an anti-PD-1 antibody and 40 µg of pro-IL-15 having the same sequences as those in the fusion proteins. The tumor size was recorded. The experimental results showed that both human-derived anti-PD-1-pro-IL-15 fusion proteins effectively controlled tumor growth in the CBMC humanized mouse model (FIG. 15).

In summary, the human-derived anti-PD-1-pro-IL-15 fusion proteins can significantly control the tumor growth in humanized mice, providing guidance for applications in cytokine engineering, bioengineering, and translational medicine.

### Example 9. anti-PD-1-Fab-IL-15-Rα-mFc and anti-PD-1-Fab-IL-15-mFc Fusion Proteins

**9.1** In this example, physically masked IL-15 was designed by using steric hindrance. IL-15 or super IL-15 (sIL-15, i.e., IL-15-Rα or Rα-IL-15) was constructed between a Fab fragment and an Fc region of an antibody to reduce the binding between IL-15 and receptor β and to avoid peripheral toxicity. Two physically masked fusion proteins were designed and prepared: anti-PD-1-Fab-IL-15-Rα-mFc and anti-PD-1-Fab-IL-15-mFc.

The functional units of each arm of the anti-PD-1-Fab-IL-15-Rα-mFc, from the N-terminus to the C-terminus, included an anti-PD-1 Fab (SEQ ID No: 1 and SEQ ID No: 2), a linker (SEQ ID No: 8), IL-15 (SEQ ID No: 16), a linking peptide (SEQ ID No: 9), an Rα sushi domain (SEQ ID No: 17), a linker (SEQ ID No: 8), and mFc (SEQ ID No: 3). The N-terminus of IL-15 was linked to the C-terminus of the heavy chain in the Fab via the linker. A structural schematic representation thereof is shown in FIG. 16.

The functional units of each arm of the anti-PD-1-Fab-IL-15-mFc, from the N-terminus to the C-terminus, included an anti-PD-1 Fab (SEQ ID No: 1 and SEQ ID No: 2), a linker (SEQ ID No: 8), IL-15 (SEQ ID No: 16), a linker (SEQ ID No: 8), and mFc (SEQ ID No: 3). The N-terminus of IL-15 was linked to the C-terminus of the heavy chain in the Fab via the linker.

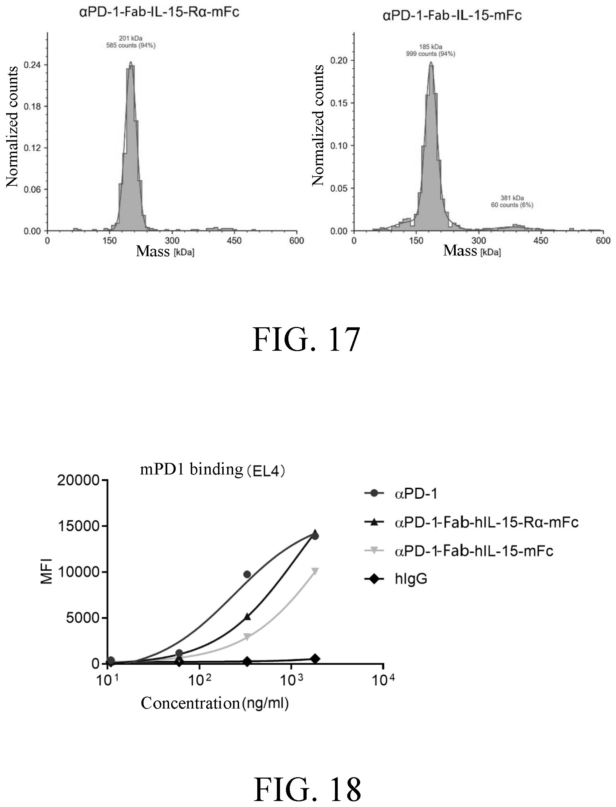

The corresponding anti-PD-1-Fab-IL-15-Rα-mFc and anti-PD-1-Fab-IL-15-mFc expression plasmids and the anti-PD-1 light-chain (SEQ ID No: 1) expression plasmid were constructed using the PEE12.4 eukaryotic expression-vector system. The anti-PD-1-Fab-IL-15-Rα-mFc and anti-PD-1-Fab-IL-15-mFc expression plasmids were separately co-transfected with the anti-PD-1 light-chain expression plasmid into 293F cells to obtain expression supernatants. The Fc-containing protein was purified by Protein A affinity chromatography, and the protein molecular size was verified by SDS-PAGE. The quality of the proteins was evaluated by mass photometry. The results showed that the proteins were of good quality and showed little aggregation (FIG. 17).

### 9.2 In Vitro Biological Activity Study of anti-PD-1-Fab-IL-15-Rα-mFc and anti-PD-1-Fab-IL-15-mFc

The binding ability of the PD-1 antibody Fab in the prepared fusion proteins to its antigen was evaluated by flow cytometry, using hIgG1 as a control. The results showed that the anti-PD-1-Fab-IL-15-Rα-mFc, anti-PD-1-Fab-IL-15-mFc, and anti-PD-1 had a similar binding ability to PD-1 molecules on the surface of EL4 cells (FIG. 18).

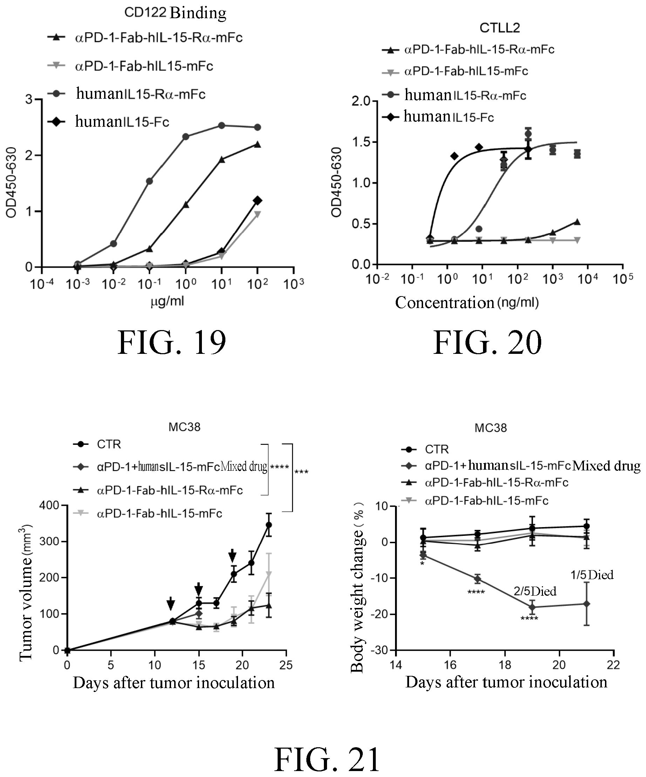

The binding ability of IL-15 or sIL-15 in the prepared fusion proteins to the IL-15 receptor β CD122 was evaluated by ELISA, using human IL-15-Rα-mFc and human IL-15-Fc as controls. The results showed that, as compared with IL-15-Rα-mFc, the binding affinity of anti-PD-1-Fab-IL-15-Rα-mFc to CD122 was reduced by approximately 5 folds, while the binding affinity of anti-PD-1-Fab-IL-15-mFc to CD122 was reduced even more (Fig. 19).

The biological activity of IL-15 in the anti-PD-1-Fab-IL-15-Rα-mFc and anti-PD-1-Fab-IL-15-mFc was evaluated by a CTLL-2 proliferation assay. The results showed that the activity of the anti-PD-1-Fab-IL-15-Rα-mFc and anti-PD-1-Fab-IL-15-mFc was significantly reduced (FIG. 20), indicating that positioning IL-15 or sIL-15 in the middle of the PD-1 antibody can substantially block IL-15 activity.

### 9.3 In Vivo Efficacy and Toxicity Study of anti-PD-1-Fab-IL-15-Rα-mFc and anti-PD-1-Fab-IL-15-mFc

The antitumor effects and toxic side effects of the anti-PD-1-Fab-IL-15-Rα-mFc and anti-PD-1-Fab-IL-15-mFc fusion proteins were evaluated in a mouse MC38 tumor model. C57BL/6 mice were subcutaneously inoculated on the back with 5×10⁵ MC38 tumor cells. On days 12, 15, and 19, the mice were each intravenously injected with (i) a blank control (CTR), or (ii) a physical mixure of 45 µg anti-PD-1 (its Fab sequence and Fc sequence were identical to those of the anti-PD-1-Fab-IL-15-Rα-mFc) and 30 µg IL-15-Rα-mFc (its sequence was identical to that of the anti-PD-1-Fab-IL-15-Rα-mFc), or (iii) 60 µg of the anti-PD-1-Fab-IL-15-Rα-mFc, or (iv) 50 µg of the anti-PD-1-Fab-IL-15-mFc. The tumor size and changes in body weight of the mice were regularly measured. The tumor growth curves and body-weight change curves were plotted.

As shown in FIG. 21, after systemic administration, the mice in the group of the physical mixture of anti-PD-1 and IL-15-Rα-mFc showed a marked decrease in body weight, and 60% of the mice died from toxicity after two administrations. In contrast, no death occurred in the anti-PD-1-Fab-IL-15-Rα-mFc or anti-PD-1-Fab-IL-15-mFc group, and the mice in these groups showed no significant body-weight loss. Both fusion proteins controlled tumor growth.

### Example 10. Design and In Vivo Antitumor Effect of anti-Lag3-pro-IL-15 Fusion Protein

In this example, an antibody targeting Lag3 was designed and linked to pro-IL-15. Similar to the PD-1 molecule, Lag3 molecule is also an immunosuppressive molecule highly expressed in tumor tissue, especially in CD8⁺ T cells in tumors. An anti-Lag3-pro-IL-15 fusion protein targeting Lag3 was constructed. Its structure was similar to that in the left panel of FIG. 1, except that the PD-1 antibody was replaced with a Lag3 antibody. The design objectives are: (1) to use the Lag3 antibody to target pro-IL-15 to CD8⁺ T cells that highly express Lag3 in tumors; (2) to block Lag3-mediated immunosuppressive signaling; and (3) to activate CD8⁺ T cells in the tumor while maintaining low peripheral toxicity by pro-IL-15, thereby controlling tumor growth.

In this example, an anti-Lag3-pro-IL-15 fusion protein was designed and constructed. The Fab of the Lag3 antibody (anti-Lag3) was the S119 clone capable of blocking Lag3 signaling; and the Fc sequence was a human IgG1 Fc sequence with three mutations (L19A, L20A, P114G), and lacked an ADCC function (designated mFc). The functional units of the fusion protein, from the N-terminus to the C-terminus, included an anti-Lag3 Fab (SEQ ID No: 18 and SEQ ID No: 19), mFc (SEQ ID No: 3), a linker (SEQ ID No: 8), an Rα sushi domain (SEQ ID No: 7), a linking peptide (SEQ ID No: 9), IL-15 (SEQ ID No: 6), an MMP-cleavable peptide (SEQ ID No: 10), and RβD1 (SEQ ID No: 4). The linker was a non-enzymatically cleavable polypeptide linker, rendering the antibody and pro-IL-15 in the complex molecule substantially non-separable *in vivo*.

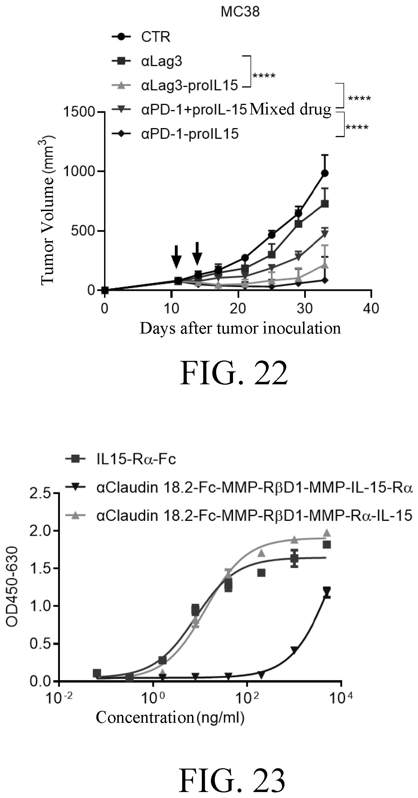

Subsequently, the antitumor effect of the anti-Lag3-pro-IL-15 was evaluated in a mouse tumor model. C57BL/6 mice were subcutaneously inoculated on the back with 5×10⁵ MC38 tumor cells. On days 11 and 14, the mice were intravenously injected with (i) a PBS blank control (CTR), or (ii) 60 µg of an anti-Lag3 antibody having the same sequence as that in the fusion protein, or (iii) 80 µg of the anti-Lag3-pro-IL-15 fusion protein, or (iv) a mixture of 52 µg of an anti-PD-1 having the same sequence as the anti-PD-1 in the anti-PD-1-pro-IL-15 fusion protein of Example 1 and 45 µg of pro-IL-15 having the same sequence as that in the fusion protein, or (v) 80 µg of an anti-PD-1-pro-IL-15 fusion protein (i.e. the non-separable anti-PD-1-pro-IL-15 fusion protein of Example 1). The results showed that the anti-Lag3-pro-IL-15 fusion protein significantly enhanced the antitumor effect of anti-Lag3 (FIG. 22).

### Example 11. Design and In Vivo Antitumor Effect of anti-Claudin18.2-pro-IL-15 Fusion Protein

**11.1** An anti-Claudin 18.2-pro-IL-15 fusion protein targeting tumor cells was designed. Claudin18.2 is expressed only in differentiated epithelial cells on the gastric mucosa and is normally not expressed in any other healthy tissue. However, Claudin18.2 is highly expressed in primary malignant tumors such as gastric cancer, breast cancer, colon cancer, and liver cancer. The design objectives of this fusion protein are: (1) to target pro-IL-15 to tumors highly expressing Claudin 18.2 by the anti-Claudin 18.2 antibody; and (2) to maintain the low peripheral toxicity of pro-IL-15 and, after the fusion protein reaches the tumor, to release active IL-15 through cleavage by an enzyme highly expressed in the tumor, thereby activating intratumoral immune responses and controlling tumor growth.

In this example, the following two releasable anti-Claudin18.2-pro-IL-15 fusion proteins were designed and constructed.

Anti-Claudin18.2-Fc-RβD1-IL-15-Rα: its functional units, from the N-terminus to the C-terminus, included an anti-Claudin18.2 Fab (SEQ ID No: 20 and SEQ ID No: 21), WT Fc (SEQ ID No: 13), a linker (SEQ ID No: 10), RβD1 (SEQ ID No: 4), an MMP-cleavable peptide (SEQ ID No: 10), IL-15 (SEQ ID No: 6), a linking peptide (SEQ ID No: 9), and an Rα sushi domain (SEQ ID No: 7). A schematic representation of its structure is shown in the right panel of FIG. 1, except that the PD-1 antibody is replaced with the Claudin18.2 antibody described above and the Fc region is not mutated or modified.

Anti-Claudin18.2-Fc-RβD1-Rα-IL-15: its functional units, from the N-terminus to the C-terminus, included an anti-Claudin18.2 Fab (SEQ ID No: 20 and SEQ ID No: 21), WT Fc (SEQ ID No: 13), a linker (SEQ ID No: 10), RβD1 (SEQ ID No: 4), an MMP-cleavable peptide (SEQ ID No: 10), an Rα sushi domain (SEQ ID No: 7), a linking peptide (SEQ ID No: 9), and IL-15 (SEQ ID No: 6). The linker was an MMP-cleavable polypeptide. Accordingly, after the complex molecule enters a tumor, the antibody portion and pro-IL-15 are separated from each other by MMP-mediated cleavage.

The corresponding anti-Claudin18.2-Fab heavy chain-pro-IL-15 expression plasmid and anti-Claudin18.2 light-chain expression plasmid were constructed using the PEE12.4 eukaryotic expression-vector system. The anti-Claudin18.2-Fab heavy chain-pro-IL-15 expression plasmid and the anti-Claudin18.2 light-chain expression plasmid were co-transfected into 293F cells to obtain an expression supernatant. The Fc-containing protein was purified by Protein A affinity chromatography, and the molecular size was verified by SDS-PAGE. The results confirmed that the two fusion proteins described above were successfully constructed.

**11.2** The biological activity of IL-15 in the above fusion proteins was evaluated by a CTLL-2 proliferation assay. The results showed that, as compared with unmasked IL-15-Rα-Fc (its sequence, from the N-terminus to the C-terminus, consisted of IL-15 (SEQ ID No: 6), a linking peptide (SEQ ID No: 9), an Rα sushi domain (SEQ ID No: 7), and WT Fc (SEQ ID No: 13)), the anti-Claudin18.2-Fc-RβD1-IL-15-Rα effectively masked the biological activity of IL-15, indicating that the IL-15 activity in the fusion protein can be masked by the receptor. However, the activity of the anti-Claudin18.2-Fc-RβD1-Rα-IL-15 was similar to that of the unmasked IL-15-Rα-Fc, indicating that, in the RβD1-Rα-IL-15 format, the IL-15 activity cannot be masked by the receptor (FIG. 23).

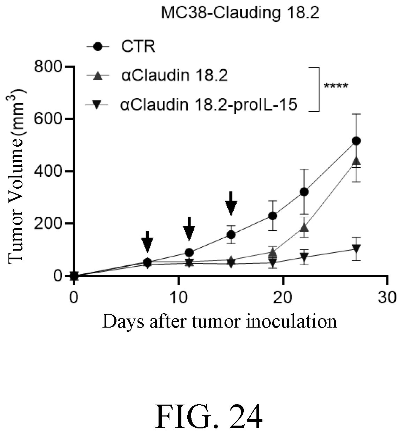

**11.3** The antitumor effect of the anti-Claudin18.2-Fc-RβD1-IL-15-Rα, whose activity can be masked, was tested in a mouse MC38 tumor model. C57BL/6 mice were subcutaneously inoculated on the back with 5×10⁵ MC38 tumor cells. On days 8, 11, and 14, the mice were intravenously injected with (i) a PBS blank control (CTR), or (ii) 60 µg of an anti-Claudin18.2 antibody having the same sequence as that in the fusion protein, or (iii) 80 µg of the anti-Claudin18.2-pro-IL-15 fusion protein. The results indicated that the anti-Claudin18.2-pro-IL-15 fusion protein significantly controlled tumor growth (FIG. 24).

Specific embodiments have been described in detail in the present specification. A person skilled in the art will appreciate that the above embodiments are merely exemplary and should not be construed as limiting the present invention. Technical solutions obtained by improvements or modifications of the present disclosure without departing from the principles of the present invention also fall within the scope of the present invention.

### References

1. Guo, Y. et al. IL-15 Superagonist-Mediated Immunotoxicity: Role of NK Cells and IFN-gamma. Journal of immunology (Baltimore, Md. : 1950) 195, 2353-2364 (2015).
2. Guo, J. et al. Tumor-conditional IL-15 pro-cytokine reactivates anti-tumor immunity with limited toxicity. Cell Res 31, 1190-1198, (2021).

## Claims

1. A complex molecule, comprising an antitumor antibody domain, a linker, and pro-IL-15, wherein the antitumor antibody domain and the pro-IL-15 are linked by the linker,
wherein the antitumor antibody domain is a complete antibody, a nanobody, or an antigen-binding fragment thereof against an immune checkpoint molecule, a tumor antigen molecule, or an immune activation molecule,
wherein the linker is a polypeptide linker or a non-peptide linker;
wherein the pro-IL-15 is a fusion protein comprising, or consisting of, an IL-15Rβ extracellular domain, IL-15, an IL-15Rα sushi domain, a first linking peptide, and a second linking peptide, wherein the IL-15Rβ extracellular domain, IL-15, and the IL-15Rα sushi domain are linked by the first linking peptide and the second linking peptide; or
the pro-IL-15 is a fusion protein comprising, or consisting of, IL-15, an IL-15Rα sushi domain, and a first linking peptide, wherein IL-15 and the IL-15Rα sushi domain are optionally linked by the first linking peptide.

2. The complex molecule according to claim 1, wherein the pro-IL-15 is
(i) a fusion protein consisting of, from the N-terminus to the C-terminus, an IL-15Rβ extracellular domain, a first linking peptide, IL-15, a second linking peptide, and an IL-15Rα sushi domain,
(ii) a fusion protein consisting of, from the N-terminus to the C-terminus, an IL-15Rα sushi domain, a second linking peptide, IL-15, a first linking peptide, and an IL-15Rβ extracellular domain,
(iii) a fusion protein consisting of, from the N-terminus to the C-terminus, IL-15, a first linking peptide, and an IL-15Rα sushi domain, or
(iv) a fusion protein consisting of, from the N-terminus to the C-terminus, a first linking peptide, IL-15, and an IL-15Rα sushi domain.

3. The complex molecule according to claim 2, wherein the first linking peptide is an MMP-cleavable peptide or a non-enzymatically cleavable peptide, and the second linking peptide is a non-enzymatically cleavable peptide.

4. The complex molecule according to any one of claims 1 to 3, wherein the immune checkpoint molecule comprises PD-1, PD-L1, CTLA4, TIGIT, Tim3, or Lag3; the tumor antigen molecule comprises CLDN18.2, EGFR, Her2, or mesothelin; and the immune activation molecule comprises OX40, 4-1BB, CD28, or CD3.

5. The complex molecule according to any one of claims 1 to 4, wherein the antitumor antibody domain is a complete antibody, a nanobody, or an antigen-binding fragment thereof against PD-1, Lag3, and/or Claudin18.2.

6. The complex molecule according to any one of claims 1 to 4, wherein the antigen-binding fragment comprises ScFv, Fab, or F(ab')₂.

7. The complex molecule according to any one of claims 1 to 6, wherein the complex molecule is a fusion protein, the linker is a polypeptide, and the complex molecule is in a form selected from:
form (a) wherein the C-terminus of at least one heavy chain of the complete antibody or nanobody is linked to the N-terminus of a pro-IL-15 via a linker;
form (b) wherein the C-terminus of an antigen-binding fragment is linked to the N-terminus of a pro-IL-15 via a linker; or
form (c) wherein the N-terminus of one chain of an Fc region is linked, via a linker, to the C-terminus of a fusion protein in form (b); and the N-terminus of the other chain of the Fc region is not linked to a peptide, or is linked, via a linker, to the C-terminus of a fusion protein in form (b), to the C-terminus of an antigen-binding fragment, or to the C-terminus of a pro-IL-15;
wherein in forms (b) and (c) the C-terminus of an antigen-binding fragment refers to the C-terminus of the heavy chain or light chain of a Fab, the C-terminus of an scFv, or the C-terminus of one heavy chain or light chain of an F(ab')₂;
wherein, when multiple linkers are present in the complex molecule, the linkers are the same as or different from each other; and when two pro-IL-15 are present in the complex molecule, the two pro-IL-15 are the same as or different from each other.

8. The complex molecule according to any one of claims 1 to 6, wherein the complete antibody or nanobody is a bispecific antibody.

9. The complex molecule according to any one of claims 1 to 8, wherein the linker is an enzymatically cleavable peptide linker or a non-enzymatically cleavable linker.

10. The complex molecule according to any one of claims 1 to 6 and 8, wherein the complex molecule is a conjugate, and the linker is a non-peptide linker.

11. The complex molecule according to any one of claims 1 to 9, wherein the linker is a peptide having an amino acid sequence of SEQ ID No: 8 or SEQ ID No: 10.

12. The complex molecule according to claim 7, wherein the complex molecule is in form (a), and the sequence of at least one heavy chain thereof, from the N-terminus to the C-terminus, consists of at least one of the following:
a heavy chain of a Fab of the antitumor antibody, an Fc, the linker, the Rα sushi domain, the second linking peptide, IL-15, the first linking peptide, and the RβD1;
a heavy chain of a Fab of the antitumor antibody, an Fc, the linker, the RβD1, the first linking peptide, IL-15, the second linking peptide, and the Rα sushi domain;
a heavy chain of a Fab of the antitumor antibody, an Fc, the linker, IL-15, the first linking peptide, and the Rα sushi domain; and
a heavy chain of a Fab of the antitumor antibody, an Fc, the first linking peptide, IL-15, and the Rα sushi domain;
wherein the antitumor antibody is an anti-PD-1 antibody, an anti-Lag3 antibody, or an anti-Claudin18.2 antibody;
wherein each heavy chain of a Fab is associated with a corresponding antibody light chain;
wherein the linker is a non-enzymatically cleavable linker, the first linking peptide is an MMP-cleavable peptide or a non-enzymatically cleavable peptide, and the second linking peptide is a non-enzymatically cleavable peptide.

13. The complex molecule according to claim 12, wherein the Fc is a wild-type Fc, or an Fc mutated or modified to have reduced or enhanced binding to FcγR.

14. The complex molecule according to claim 12, wherein the Fc has a normal ADCC function, has no ADCC function, or has an enhanced ADCC function.

15. The complex molecule according to claim 12, wherein the Fc having no ADCC function has a sequence of SEQ ID No: 3, and the Fc having a normal ADCC function has a sequence of SEQ ID No: 13.

16. The complex molecule according to claim 6 or 12, wherein the Fab is at least one selected from:
(i) a Fab having a heavy chain sequence of SEQ ID No: 2 or SEQ ID No: 12, and a light chain sequence of SEQ ID No: 1 or SEQ ID No: 11;
(ii) a Fab having a heavy chain sequence of SEQ ID No: 19, and a light chain sequence of SEQ ID No: 18;
(iii) a Fab having a heavy chain sequence of SEQ ID No: 21, and a light chain sequence of SEQ ID No: 20.

17. The complex molecule according to any one of claims 1 to 16, wherein in the pro-IL-15, IL-15 has a sequence of SEQ ID No: 6 or SEQ ID No: 16, the IL-15Rα sushi domain has a sequence of SEQ ID No: 7 or SEQ ID No: 17, the IL-15Rβ extracellular domain has a sequence of SEQ ID No: 4, SEQ ID No: 14, SEQ ID No: 5, or SEQ ID No: 15, the first linking peptide has a sequence of SEQ ID No: 8, SEQ ID No: 9, or SEQ ID No: 10, and the second linking peptide has a sequence of SEQ ID No: 8 or SEQ ID No: 9.

18. A complex molecule obtained by inserting pro-IL-15 or IL-15 between the CH1 and CH2 of at least one heavy chain of a complete antitumor antibody,
wherein linkers are optionally linked to the N-terminus and C-terminus of the pro-IL-15 or IL-15;
wherein the pro-IL-15 is a fusion protein consisting of, from the N-terminus to the C-terminus, IL-15, a linking peptide, and an IL-15Rα sushi domain, or an IL-15Rα sushi domain, a linking peptide, and IL-15;
wherein the antitumor antibody is an antibody against an immune checkpoint molecule, a tumor antigen molecule, or an immune activation molecule,
wherein the linkers are each a polypeptide linker or a non-peptide linker; and
wherein the linking peptide is an enzymatically cleavable or non-enzymatically cleavable peptide.

19. The complex molecule according to claim 18, wherein the complex molecule is a fusion protein in a form in which one heavy chain consists of, from the N-terminus to the C-terminus, a heavy chain of a Fab of the antitumor antibody, a linker, pro-IL-15/IL-15, a linker, and one chain of an Fc; and the other heavy chain consists of, from the N-terminus to the C-terminus, a heavy chain of a Fab of an antitumor antibody, a linker, pro-IL-15/IL-15, a linker, and the other chain of the Fc;
wherein the two heavy chains of the Fabs are the same as or different from each other and are each associated with a corresponding antibody light chain to form a Fab, the two chains of the Fc are associated with each other through a disulfide bond, the linkers are the same as or different from one another, and the two pro-IL-15 are the same as or different from each other.

20. The complex molecule according to claim 19, wherein the Fab is selected from the Fabs as defined in claim 16; the linker is selected from SEQ ID No: 8, SEQ ID No: 9, or SEQ ID No: 10; the IL-15 is selected from SEQ ID No: 6 or SEQ ID No: 16; the IL-15Rα sushi domain is selected from SEQ ID No: 7 or SEQ ID No: 17; the linking peptide is selected from SEQ ID No: 8 or SEQ ID No: 9; and the Fc is the Fc as defined in any one of claims 13 to 15.

21. The complex molecule according to claim 20, wherein the linker linked to the heavy chain of a Fab is SEQ ID No: 8 or SEQ ID No: 9, and the linker linked to the Fc is SEQ ID No: 10.

22. A nucleic acid encoding the complex molecule according to any one of claims 7, 12-15, and 17-21.

23. A vector comprising the nucleic acid according to claim 22.

24. A host cell comprising the vector according to claim 23.

25. A pharmaceutical composition, comprising:
the complex molecule according to any one of claims 1 to 21, the nucleic acid according to claim 22, the vector according to claim 23, or the host cell according to claim 24, and
a pharmaceutically acceptable carrier or excipient.

26. The pharmaceutical composition according to claim 25, wherein the antitumor antibody domain is a complete antibody, a nanobody, or an antigen-binding fragment thereof against PD-1; preferably, wherein the pharmaceutical composition further comprises an anti PD-L1 antibody.

27. Use of the complex molecule according to any one of claims 1 to 21, the nucleic acid according to claim 22, the vector according to claim 23, the host cell according to claim 24, or the pharmaceutical composition according to claim 25 or 26 in the manufacture of a medicament for treating cancer, preferably a solid tumor.

28. The use according to claim 27, wherein the cancer is selected from the group consisting of lung cancer, liver cancer, gastric cancer, cervical cancer, ovarian cancer, breast cancer, colorectal cancer, melanoma, and lymphoma.
